# EUROPEAN PATENT SPECIFICATION

(11) **EP 2 300 616 B1**
(45) Date of publication and mention of the grant of the patent: **26.04.2017**
(21) Application number: 09761991.0
(22) Date of filing: 12.06.2009
(51) Int. Cl.: C12N 15/82

(54) **SELECTION METHOD II**
SELEKTIONSVERFAHREN II
PROCÉDÉ DE SÉLECTION II

(30) Priority: 13.06.2008 US 61405 P
(43) Date of publication of application: 30.03.2011
(73) Proprietor: Plastid As, 4068 Stavanger (NO)
(72) Inventor: MOLLER, Simon, Geir, Stavanger (NO); CHUA, Nam-Hai, New York, NY 10021 (US); MAPLE, Jodi, Stavanger (NO)
(74) Representative: Webber, Philip Michael
(86) International application number: PCT/GB2009/001511
(87) International publication number: WO 2009/150442

(56) References cited:
- WO-A-2008/071973
- US-A1- 2006 253 916
- S.G. MOLLER: "Chloroplast genetic engineering for crop improvement and production of high value compounds" PROCEEDINGS OF THE FIRST INTERNATIONAL WORKSHOP ON GROWING PLANTS FOR INCREASED NUTRICIONAL VALUE, 12 May 2005 (2005-05-12), - 14 May 2005 (2005-05-14) page 21, XP002546146 University of Stavanger, Norway
- ENDO S ET AL: "A new GST-MAT vector containing both ipt and iaaM/H genes can produce marker-free transgenic tobacco plants with high frequency" PLANT CELL REPORTS, vol. 20, no. 10, March 2002 (2002-03), pages 923-928, XP002546102 ISSN: 0721-7714
- LUTZ ET AL: "Construction of marker-free transplastomic plants" CURRENT OPINION IN BIOTECHNOLOGY, LONDON, GB, vol. 18, no. 2, 17 April 2007 (2007-04-17), pages 107-114, XP022032088 ISSN: 0958-1669
- LUTZ KERRY A ET AL: "Plastid marker-gene excision by transiently expressed CRE recombinase" PLANT JOURNAL, vol. 45, no. 3, February 2006 (2006-02), pages 447-456, XP002546103 ISSN: 0960-7412
- CORNEILLE S ET AL: "Efficient elimination of selectable marker genes from the plastid genome by the CRE-lox site-specific recombination system", THE PLANT JOURNAL, BLACKWELL SCIENTIFIC PUBLICATIONS, OXFORD, GB, vol. 27, no. 2, 1 July 2001 (2001-07-01), pages 171-178, XP002236641, ISSN: 0960-7412, DOI: 10.1046/J.1365-313X.2001.01068.X
- HAJDUKIEWICZ P T J GILBERTSON L STAUB J M: "Multiple pathways for Cre/lox-mediated recombination in plastids", THE PLANT JOURNAL, BLACKWELL SCIENTIFIC PUBLICATIONS, OXFORD, GB, vol. 27, no. 2, 1 January 2001 (2001-01-01), pages 161-170, XP002959442, ISSN: 0960-7412, DOI: 10.1046/J.1365-313X.2001.01067.X

## Description

The invention relates to a method for producing a transformed plant cell. More particularly, the method involves the transformation of a plant cell with a Transformation Cassette which is targeted to plant plastids and which comprises a selection gene, and a transgene. After selection for transformed plastids, expression of a recombinase is induced in the plant cell, which leads to the excision of the selection gene from the plastid and the expression of the transgene in the plastid. The invention also provides cells and plants comprising the Transformation Cassette.

The use of genetically modified (GM) food crops in agriculture is rapidly increasing with an approximate £14 billion world market in 2005. There is, however, a growing concern amongst scientists, politicians, regulatory agencies and the general public regarding the risk of the spread of transgenes by pollen from GM crops to other plant species generating so-called "superweeds".

All commercial GM crops used in today's agriculture have engineered nuclear genomes containing transgenes conferring desirable traits such as resistance to disease, insects, harsh environmental conditions and increased vitamin content, flavour, and storage time. However, there are several serious problems with nuclear transgenes, including gene silencing and low levels of transgene expression. One way of resolving the problems associated with nuclear transgenes is to insert transgenes into the plastid genome of plants.

Plastids are organelles unique to plants. Each plant cell contains approximately 100 plastids each of which contains approximately 100 genomes. This in effect means that when a gene is inserted into the plastid genome each plant cell contains approximately 10,000 copies of that gene compared to plant nuclear transformation where each plant cell would have at best 2-3 copies of the gene.

The expression of transgenes in plastids has numerous advantages over nuclear gene expression: (i) transgenes are not spread by pollen; (ii) proteins are expressed to high levels (up to 47% of total cellular protein); (iii) "toxic" effects of proteins are reduced due to plastid containment; (iv) the simultaneous expression of several genes allows for biochemical pathway engineering; and (v) gene silencing is eliminated. Plastid genetic engineering has clear fundamental and applied applications in that potentially any protein can be produced to high levels with little environmental risk opening up the possibility of producing edible vaccines, biopharmaceuticals and products of agronomical value.

Currently, the only reliable selection systems that are used to select for plastids, which have been transformed with transgenes, are based on the use of antibiotics such as spectinomycin. A disadvantage of such selection systems is that they will also select for spontaneous ribosomal mutants which are resistant to spectinomycin. This is a large problem, given the time-scales involved.

The invention is based on a selection and regeneration system for plastid transformation based on the over-expression of a gene in plastids. The system therefore provides an antibiotics-free selection and regeneration system which will overcome problems with spontaneous spectinomycin mutants and concerns regarding the use of antibiotic resistance genes in GMOs.

IPT has previously been used as a selectable marker in plant nuclear transformation (e.g. EP 1 069 855 A) and in plastid transformation (S.G. Moller: "Chloroplast genetic engineering for crop improvement and production of high value compounds",Proceedings of the first international workshop on growing plants for increased nutricional value, 12 May 2005, page 21, University of Stavanger, Norway)

Due to the fact that over-expression of plant-hormone biosynthetic polypeptides in adult plants leads to impaired development, the plant-hormone biosynthetic gene is preferably removed after selection and initial regeneration has occurred. The transgene in question (expressing the polypeptide of interest) might only be activated after the removal of the plant-hormone biosynthetic gene, so that any adverse effects due to transgene expression during selection and regeneration are also eliminated.

In one embodiment, the invention provides a method for producing a transformed plant cell, the method comprising the step:
(i) transforming the plant cell with a genetic construct, wherein the genetic construct comprises first and second homologous recombination elements flanking a Transformation Cassette, wherein the first and second homologous recombination elements are capable of directing the integration of the Transformation Cassette into the genome of at least one plastid which is present in the plant cell, wherein the Transformation Cassette comprises:
   (a) a first promoter which is operable in said plant cell,
   (b) an Excision Cassette,
   (c) one or more transgenes,
   (d) a first terminator element,
   wherein the Excision Cassette comprises:
   (b1) a first site-specific recombination element,
   (b2) an optional second promoter
   (b3) one or more nucleotide sequences encoding one or more plant-auxin biosynthetic polypeptides and wherein the plant-auxin biosynthetic polypeptides are selected from iaaH (indoleacetamide hydrolase) and iaaM (tryptophan mono-oxygenase) and optionally a nucleotide sequence encoding a polypeptide which confers resistance to an antibiotic,
   (b4) a second terminator element,
   (b5) a second site-specific recombination element,
   wherein the first and second site-specific recombination elements are lox sites and are capable of being recognised by a recombinase, wherein the recombinase is a Cre recombinase.

In some embodiments, the method additionally comprises the step:
(ii) selecting for transformed plant cells on media which lacks auxin.

In other embodiments, the method additionally comprises the step:
(iii) expressing a recombinase in the plant cell, wherein the recombinase is one which recognises the first and second site-specific recombination elements.

The method of the invention is suitable for all plants that can be transformed and regenerated. The plant may be a monocot or dicot.

Examples of suitable plants are cereals (rice, wheat, barley, oats, sorghum, corn), legumes (alfalfa, lentils, peanut, pea, soybean), oil crops (palm, sunflower, coconut, canola, olive), cash crops (cotton, sugar cane, cassava), vegetable crops (potato, tomato, carrot, sweet potato, sugar-beet, squash, cucumber, lettuce, broccoli, cauliflower, snap bean, cabbage, celery, onion, garlic), fruits/trees and nuts (banana, grape cantaloupe, muskmelon, watermelon, strawberry, orange, apple, mango, avocado, peach, grapefruit, pineapple, maple, almond), beverages (coffee, tea, cocoa), and timber trees (oak, black walnut, sycamore). Other suitable plants include mosses and duckweed. Preferably, the plant is tobacco or lettuce.

The plant cells which are being transformed may be used in any convenient form, for example, as individual cells, groups of cells, in dissociated form or undissociated form, or as part of a plant tissue or plant part. Preferably, the cells are present in leaves that are removed from intact plants. It is preferable to use actively-growing leaves.

The term "plastid" is intended to cover all organelles which are found in the cytoplasm of eukaryotic plants, which contain DNA, which are bounded by a double membrane, and develop from a common type, i.e. a proplastid. Plastids may contain pigments and/or storage materials.

Examples of plastids include chloroplasts, leucoplasts, amyloplasts, etioplasts, chromoplasts, elaioplasts and gerontoplasts. Preferably, the plastid is a green plastid, most preferably a chloroplast.

As used herein, the term "genetic construct" refers to a nucleic acid molecule comprising the specified elements and Cassettes. The genetic construct may, for example, be in the form of a vector or a plasmid. It may also contain other elements which enable its handling and reproduction, such as an origin of replication, selection elements, and multiple cloning sites. Generally, the genetic construct will be a double-stranded nucleic acid molecule, preferably a dsDNA molecule.

The first and second homologous recombination elements are ones that are capable of directing the integration of the Transformation Cassette into the genome of at least one plastid which is present in the plant cell.

Upon transformation of the genetic construct into the plant cell, the first and second homologous recombination elements recombine with corresponding sequences in the genome of the selected plastid or plastids, resulting in the insertion of the Transformation Cassette into the genome of the selected plastid or plastids.

The nucleotide sequences of the homologous recombination elements are selected such that the Transformation Cassette is specifically targeted to one or more selected plastids. In particular, the nucleotide sequences of the homologous recombination elements are selected such that no or essentially no Transformation Cassettes become integrated into the nuclear genome of the plant or into the mitochondrial genome of the plant. In other words, the nucleotide sequences of the homologous recombination elements are preferably plastid-specific, i.e. corresponding sequences are not present in the nuclear genome and preferably not present in the mitochondrial genome of the plant in question. This may be done by avoiding sequences which are present in the nuclear genome of the plant and optionally in the mitochondrial genome. The skilled person will readily be able to detect whether a specific sequence is or is not present in the nuclear genome by standard means, for example, by Southern Blotting of the nuclear genome with a labelled sequence probe or by sequence analysis.

Apart from the above, any sequences can be used from the plastid genome as long as the selected insertion site is not lethal to the cell, i.e. it does not result in the death of the cell. Preferably, the insertion sites are not in coding regions of plastid genes.

The orientation of the sequences of the first and second homologous recombination elements should be the same as the orientation in the plastid genome to allow for efficient homologous recombination.

In order to target the Transformation Cassette to the plastid genome, the nucleotide sequences of the first and second homologous recombination elements must be identical or substantially identical to sequences in the genome of the selected plant plastid.

In the context of the present invention, the term "substantially identical" means that the nucleotide sequences of the first and second homologous recombination sequences are independently more than 95%, preferably more than 98% or more than 99% and particularly preferably 100% identical to sequences which are present in the plastid to be transformed. Percentage sequence identities may be determined using the Clustal method of alignment with default parameters, e.g. KTUPLE 1, GAP PENALTY=3, WINDOW=5 and DIAGONALS SAVED =5.

Similarly, the nucleotide sequences of the first and second homologous recombination elements should preferably not be identical or substantially identical to sequences in the nuclear genome of the selected plant. In this context, the term "substantially identical" means that the nucleotide sequences of the first and second homologous recombination sequences are independently less than 50%, more preferably less than 70% or less than 90% identical to sequences which are present in the nuclear genome of the plant to be transformed. Percentage sequence identities may be determined using the Clustal method of alignment with default parameters, e.g. KTUPLE 1, GAP PENALTY=3, WINDOW=5 and DIAGONALS SAVED=5.

Preferably, the lengths of first and second homologous recombination sequences will independently be 50-2500, 50-2000, 50-1500 or 50-1000 nucleotides each, more preferably about 150, about 1000 or about 1200 nucleotides in length.

The distance between the first and second homologous recombination sequences in the plastid genome may be 0-4000 nucleotides or more. Preferably, the distance is about 1-100, 100-500, 500-1000 or 1000-3000 nucleotides.

The total length of the genetic elements which are present between the first and second homologous recombination (i.e. genetic elements (a)-(d)) is preferably less than 4000 nucleotides.

Preferably, the first homologous recombination sequence is nucleotides 104091-105380 of the *Nicotiana tabacum* (accession no. Z00044) chloroplast genome DNA; and/or preferably, the second homologous recombination sequence is nucleotides 105381-106370 of the *Nicotiana tabacum* (accession no. Z00044) chloroplast genome DNA.

In yet other embodiments, the first homologous recombination sequence is preferably nucleotides 102925-101857 of the *Nicotiana tabacum* (accession no. Z00044) choloroplast genome DNA; and/or the second homologous recombination sequence is nucleotides 100933-100130 of the *Nicotiana tabacum* (accession no. Z00044) choloroplast genome DNA.

The Transformation Cassette promoter (a) must be one that is operable in the selected plant plastid. The promoter is one which is capable of initiating transcription of the transgene once the Excision Cassette has been excised; and of initiating the transcription of the nucleotide sequence encoding the plant-auxin biosynthetic polypeptide(s), in cases where the Excision Cassette does not contain its own promoter. The promoter might, for example, be one derived from a plant or bacterial gene. Preferably, the promoter is plant specific.

Examples of suitable promoters include PpsbA, RbcL and Prrn promoters.

Preferably, the promoter is a Prrn promoter (e.g. Plastidic ribosomal RNA (rrn) operon promoter (nt 59034-59303, accession Z00044 *Nicotiana tabacum* chloroplast genome DNA).

Other preferred promoters include the Prrn-rbcl-ds promoter construct as shown in Figure 24 and the legend thereto; the Prrn-atpB-ds promoter construct as shown in Figure 25 and the legend thereto; and the Prrn-T7g10 promoter construct as shown in Figure 26 and the legend thereto.

In some embodiments, the promoter is an inducible promoter. This allows inducible, controlled expression of the selection gene(s). For example, the inducible promoter may be inducible by IPTG, e.g. the PrrnL promoter. Other inducible promoters include those inducible by light, dark, ethanol, drought, metals, pathogens, growth regulators, heat, cold, galactose and other sugars. Alternatively, the promoter is a high-expression level promoter.

The Excision Cassette comprises a first site-specific recombination element; optionally, a second promoter; a nucleotide sequence encoding the plant-auxin biosynthetic polypeptide(s); a terminator sequence; and a second site-specific recombination element, wherein the first and second site specific recombination elements are lox sites.

The first and second site-specific recombination elements are sequences of nucleotides which are capable of being recognised and/or bound by the site-specific recombinase which is produced by a recombinase. The site-specific recombination elements must flank the genetic elements in the Excision Cassette, e.g. elements (b2) (if used), (b3), (b4), any other desired elements. The sequences of the first and second recombination elements will be identical or substantially identical to each other; and will be in the same orientation relative to each other (e.g. both 5'-3' or both 3'-5').

The recombinase is Cre; the site-specific recombination sequences are lox sequences. Site-specific lox recombination sites are 34 bp sequences; these act as binding sites for the Cre recombinase polypeptide. Wild-type lox sequences are preferred (Zuo J, Niu QW, Møller SG, Chua NH (2001) Chemical-regulated, site-specific DNA excision in transgenic plants. Nat. Biotechnol. 19, 157-161.)

The Excision Cassette promoter, when present, must be one that is operable in the selected plant plastid. The promoter is one which is capable of initiating transcription of the plant-auxin biosynthetic gene. The promoter might be one derived from a plant or bacterial gene. Preferably, the promoter is plant specific. Examples of suitable promoters include PsbA, RbcL and Prrn promoters. Preferably, the promoter is a Prrn promoter (e.g. Plastidic ribosomal RNA (rrn) operon promoter (nt 59034-59303, accession Z00044 *Nicotiana tabacum* chloroplast genome DNA).

The plant-auxin biosynthetic polypeptides act as selection markers, allowing the selection of plant cells which have been transformed with the Transformation Cassette.

The nucleotide sequences encoding the plant-auxin biosynthetic polypeptides may be present in an operon, with a single optional promoter and terminator element. Alternatively, the plant-auxin biosynthetic polypeptide nucleotide sequences may each have their own promoters and terminator elements. A further option is that two or more of the nucleotide sequences encoding the plant-auxin biosynthetic polypeptides are present as fusion proteins, optionally with a short linker sequence joining the proteins (e.g. encoding a 1-10 amino acid linker sequence, e.g. a poly-glycine linker). In other embodiments, some of the nucleotide sequences encoding the plant-auxin biosynthetic polypeptides may be present in an operon and/or as fusion proteins, and others have their own promoters and/or terminators.

The plant-auxin biosynthetic polypeptide is iaaH (indoleacetamide hydrolase) and/or iaaM (tryptophan mono-oxygenase), which are enzymes involved in auxin biosynthesis. The nucleotide sequences may be from any source. Due to codon usage, bacterial iaaH and/or iaaM genes are preferred.

Preferably, the iaaH and/or iaaM nucleotide sequences are from *Agrobacterium tumefaciens.*

In other embodiments of the invention where auxin biosynthetic enzymes are involved, the transformed plants may be selected on media lacking auxins. Examples of naturally-occurring auxins include 4-chloro indoleacetic acid, phenyl acetic acid (PAA) and indole-3-butyric acid (IBA).

The plant auxin biosynthetic enzymes are iaaH and iaaM, preferably from *Agrobacterium tumefaciens.*

The Excision Cassette terminator prevents the premature expression of the transgene(s) prior to the excision of the Excision Cassette. Any terminator can be used for this provided that it is recognised in the plant cell being transformed. The terminator may be a plant terminator or a bacterial terminator, *inter alia.*

Examples of suitable terminators include those of rrn, psbA, rbcL and T7.

The preferred terminator is a TrbcL terminator (e.g. Ribulose-1,5-Bisphosphate Carboxylase/Oxygenase polyA addition sequence (nt 102539-102685, accession Z00044 *Nicotiana tabacum* chloroplast genome DNA).

In some embodiments of the invention, the promoter and terminator used in the Excision Cassette do not both originate from the same plastid gene.

In the context of the present invention, the term "transgene" (i.e. element (c)) is used to refer to a nucleic acid molecule which is being introduced into the genome of the plastid. The transgene may, for example, be a genomic DNA, cDNA or synthetic nucleic acid molecule coding for a peptide or polypeptide; a nucleic acid molecule encoding a mRNA, tRNA or ribozyme; or any other nucleic acid molecule.

Examples of transgenes include those coding for antibodies, antibiotics, herbicides, vaccine antigens, enzymes, enzyme inhibitors and design peptides.

Single or multiple antigens may be produced from viridae, bacteria, fungi or other pathogens. The antigens may be expressed as single units or as multiple units of several antigens, e.g. for broad-spectrum vaccine development.

Enzymes may be produced for use in cosmetics (e.g. superoxide dismutase, peroxidase, etc.). Enzymes may also be produced for use in detergent compositions.

Proteins/enzymes with specific activities may be produced, for example, immunostimulants to boost immune responses, such as interferons; and growth factors, e.g. transforming growth factor-beta (TGF-beta), bone morphogenic protein
(BMP), neurotrophins (NGF, BDNF, NT3), fibroblast growth factor (FGF), proteolytic enzymes (papain, bromelain), and food supplement enzymes (protease, lipase, amylase, cellulase).

Proteins/enzymes may be produced or overexpressed in plastids that make the plants more resistant to biotic and abiotic stress, such as salts and metals. Examples of this include the generation of transplastomic plants that chelate iron (Fe) for mopping up excess metal in agriculturally important areas for future planting.

One or more transgenes may be inserted in the Transformation Cassette. Preferably, the transgene sequences are contiguous.

The transgene sequence may additionally encode a protein purification tag fused to the polypeptide of interest. Examples of protein purification tags include the N-terminal influenza haemagglutinin-HA-epitope (HA) and a sequence of six histidine amino acids (HIS6) and the Strep tag. Each of the transgene products may have a different affinity tag.

Once the Excision Cassette has been excised, the first promoter is capable of driving the expression of the transgene, leading to the accumulation of the product of the transgene in the plastid. The product of the transgene may be purified or isolated from the plant cell by any suitable means.

In some embodiments of the invention, the transgene is constitutively expressed independently of whether or not the Excision Cassette has been removed. In such embodiments, the transgene may be expressed from its own promoter. Such embodiments are useful if the plant does not show any detrimental effects of the transgene expression during regeneration.

The Transformation Cassette terminator (genetic element (d)) terminates the expression of the transgene(s). Any terminator can be used for this provided that it is recognised in the plant cell being transformed. The terminator may be a plant terminator or a bacterial terminator, *inter alia.*

Examples of suitable terminators include TrbcL or TspbA polyA addition sequences. The preferred terminator is the *psbA* polyA addition sequence.

In the Transformation Cassette, the elements are preferably operably linked in the order (a), (b), (c), (d).

In the Excision Cassette, the first and second site-specific recombination elements must flank the optional promoter (when present), the nucleotide sequence encoding a plant-auxin biosynthetic polypeptide and the terminator element.

In some embodiments of the invention, the nucleotide sequence encoding a plant-auxin biosynthetic polypeptide and the terminator element will be downstream (i.e. 3') to the promoter of the Transformation Cassette and hence the latter promoter will be capable of driving expression of the plant-auxin biosynthetic polypeptide.

Thus in some embodiments of the invention, the Transformation Cassette comprises:
(a) a first promoter which is operable in said plant cell,
(b) an Excision Cassette comprising:
   (b1) a first site-specific recombination element,
   (b3) one or more nucleotide sequences encoding one or more
      plant-auxin biosynthetic polypeptides, wherein the plant-auxin biosynthetic polypeptides are selected from iaaH (indoleacetamide hydrolase) and iaaM (tryptophan mono-oxygenase),
   (b4) a second terminator element,
   (b5) a second site-specific recombination element,
      wherein the first and second site-specific recombination elements are lox sites and are capable of being recognised by a recombinase, where the recombinase is a Cre recombinase;
(c) one or more transgenes,
(d) a first terminator element,
operably linked in the order specified above in a 5'-3' direction.

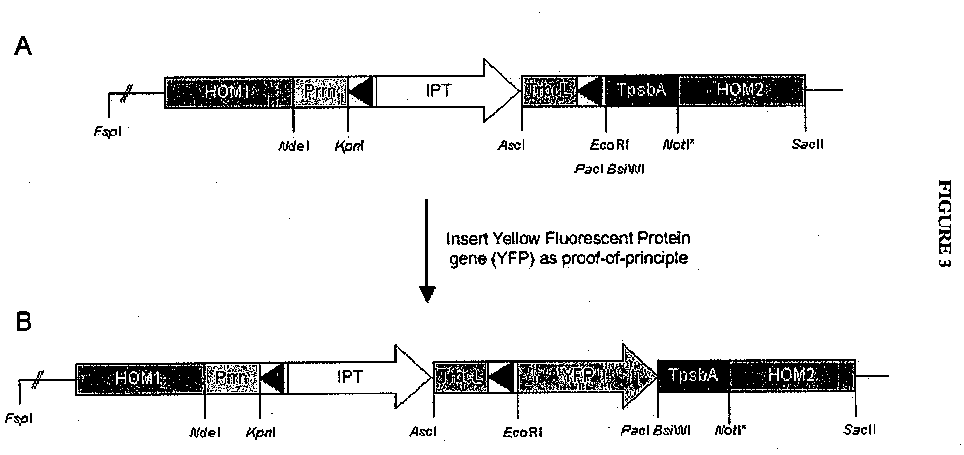

In this embodiment of the invention, the first promoter is capable of driving expression of the nucleotide sequence encoding the plant-auxin biosynthetic polypeptides (for example, as shown in Figures 3-4). After the removal of the Excision Cassette, the first promoter drives expression of the transgene(s).

In other embodiments of the invention, the Excision Cassette will be in the reverse orientation compared to the first promoter, transgene(s) and first terminator element. In this embodiment, the expressed parts of the Excision Cassette will be present in the nucleotide strand which is complementary to that which codes for the first promoter, transgene(s) and first terminator element, and in the reverse direction.

In such embodiments, the Excision Cassette will comprise a second promoter, capable of driving the expression of the nucleotide sequences encoding the plant-auxin biosynthetic polypeptides.

In this embodiment of the invention, the Transformation Cassette comprises:
(a) a first promoter which is operable in said plant cell,
(b) an Excision Cassette,
(c) one or more transgenes,
(d) a first terminator element,
wherein (a), (b), (c) and (d) are operably linked in the order specified above in a 5'-3' direction,
wherein the Excision Cassette comprises:
(b1) a first site-specific recombination element,
(b2) a second promoter,
(b3) one or more nucleotide sequences encoding one or more plant-auxin biosynthetic polypeptides, wherein the plant-auxin biosynthetic polypeptides are selected from iaaH (indoleacetamide hydrolase) and iaaM (tryptophan mono-oxygenase),
(b4) a second terminator element,
(b5) a second site-specific recombination element,
wherein the first and second site-specific recombination elements are lox sites and are capable of being recognised by a recombinase, wherein the recombinase is a Cre recombinase and wherein the parts of the Excision Cassette are operably linked and wherein the Excision Cassette is in reverse orientation compared to (a), (c) and (d).

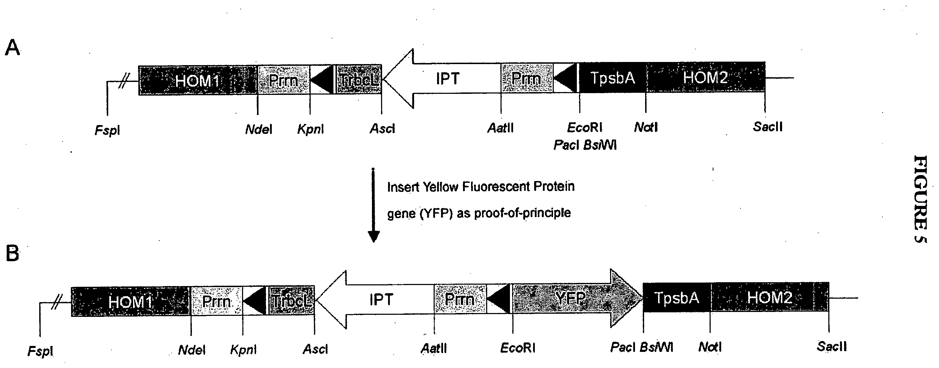

In this embodiment, the second promoter drives expression of the nucleotide sequences encoding the plant-auxin biosynthetic polypeptides (for example, as shown in Figures 5-6). After the removal of the Excision Cassette, the first promoter drives expression of the transgene(s).

The Transformation Cassette is not restricted to the parts (a)-(d) specified herein. It may, for example, additionally comprise a 5'-UTR to increase the expression level of the transgene(s) and/or 3'-additional amino acids to increase protein stability.

In some embodiments of the invention, the Transformation Cassette additionally comprises a second selectable marker gene, e.g. an antibiotic resistance gene, preferably a nucleotide sequence encoding spectinomycin adenyltransferase (e.g. the aadA gene). This polypeptide confers resistance to the antibiotic spectinomycin. The nucleotide sequence enoding spectinomycin adenyltransferase may be placed downstream of one of the promoters in the Transformation Cassette. It may, for example, be placed downstream and operably linked to the first promoter; or downstream and operably linked to the nucleotide sequences encoding the plant-auxin biosynthetic polypeptides; or upstream and operably linked to the nucleotide sequences encoding the plant-auxin biosynthetic polypeptides.

In yet other embodiments, the Transformation Cassette excludes a second selectable marker gene and/or excludes a nucleotide sequence which confers resistance to an antibiotic.

In some embodiments, the Transformation Cassette additionally comprises the LacI gene, preferably under control of an appropriate promoter (e.g. T7), in order to allow for inducible expression.

After successful delivery of the Transformation Cassette to the plastid(s), transformed cells are selected on media lacking auxin. In general, plants are regenerated by adding cytokinin and auxin. The cells for selection will preferably be leaf cells.

In some embodiments of the invention, plants are selected in the presence of cytokinin inhibitors (e.g. cytokinin oxidase or chemical inhibitors) in order to minimise leakage of the cytokinin from the site of biosynthesis to other parts of the plant. This reduced mosaicism in the plant.

In some embodiments of the invention, the method additionally comprises the step:
(iii) expressing a recombinase in the plant cell, wherein the recombinase is one which recognises the first and second site-specific recombination elements.

The recombinase is a Cre site-specific recombinase. A site-specific recombinase is a polypeptide which is capable of binding to site-specific recombination elements and inducing a cross-over event in the nucleic acid molecule in the vicinity of the site-specific recombination elements. In the present invention, the expression of the recombinase leads to the excision of the Excision Cassette from the plastid genome.

In the context of the present invention, the recombinase is one which is capable of binding to the first and second site-specific recombination elements which are present in the Excision Cassette, leading to the excision of the Excision Cassette in a standard manner.

Examples of site-specific recombination elements/site-specific recombinases include Cre-lox, the FLP-FRP system from *Saccharomyces cerevisae* (O'Gorman S, Fox DT, Wahl GM. (1991) Recombinase-mediated gene activation and site-specific integration in mammalian cells. Science. 25, 1351-1555.), the GIN/gix system from bacteriophage Mu (Maeser S and Kahmann R. (1991) The Gin recombinase of phage Mu can catalyse site-specific recombination in plant protoplasts. Mol Gen Genet. 230, 170-176.) or the R/RS system from *Zygosaccharomyces rouxii* (Onouchi H, Yokoi K, Machida C, Matsuzaki H, Oshima Y, Matsuoka K, Nakamura K, Machida Y. (1991) Operation of an efficient site-specific recombination system of Zygosaccharomyces rouxii in tobacco cells. Nucleic Acids Res. 19, 6373-6378.).

The nucleotide sequence which codes for the recombinase may comprise an intron, preferably a plant-specific intron. The presence of such an intron will suppress the expression of the recombinase polypeptide in prokaryotes, for example bacteria.

The recombination site is lox in combination with the recombinase Cre. Preferably, the recombinase sequence used is a cDNA sequence encoding a Cre polypeptide.

Preferably, all or substantially all of the Excision Cassettes are excised from the plastid genome by the recombinase. The skilled person will understand, however, that some or all of the sequences of one or more recombination elements might remain in the plastid genome. The recombinase may be expressed in the cell by any suitable means.

In some embodiments of the invention, the plant cell is one which already comprises an expressible construct which is integrated into the nuclear genome, wherein the expressible construct comprises a nucleotide sequence encoding a plastid-targeting transit peptide and a recombinase (operably linked, i.e. in frame). The expressible construct might, for example, have been introduced into the nuclear genome by homologous recombination. In such cases, the recombinase must be under the control of an inducible promoter. Such an inducible promoter may have been introduced with the construct or the construct may have been integrated adjacent to an endogenous inducible promoter.

Plants containing nuclear-located sequences encoding recombinases may be removed from a desired population by crossing, wherein the sequences may be lost due to segregation of this trait.

In other embodiments of the invention, the plant cell is one which already comprises an expressible construct which is integrated into the genome of the desired plastid(s), wherein the expressible construct comprises a nucleotide sequence encoding a recombinase. The expressible construct might, for example, have been introduced into the plastid genome by homologous recombination. In such cases, the recombinase must be under the control of an inducible promoter. Such an inducible promoter may have been introduced with the construct or the construct may have been integrated adjacent to an endogenous inducible promoter.

Thus in some embodiments of the invention, step (iii) comprises:
(iii) inducing the expression of a recombinase in the plant cell from an inducible promoter operably linked to a nucleotide sequence encoding a recombinase which is present in the plant cell, wherein the recombinase recognises the first and second site-specific recombination elements.

Preferably, the inducible promoter operably linked to a nucleotide sequence encoding a recombinase is present in the nuclear genome of the plant cell.

In other preferred embodiments, the inducible promoter operably linked to a nucleotide sequence encoding a recombinase is present in a plastid genome of the plant cell.

In yet other embodiments of the invention, the plant cell is transformed with a Recombinase Vector which comprises a promoter operably linked to a nucleotide sequence encoding a recombinase, either before step (i), simultaneously with step (i) or after step (i); or before step (ii), simultaneously with step (ii) or after step (ii).

The Recombinase Vector is a nucleic acid vector that comprises a promoter element that is capable of driving the expression of a downstream recombinase. The vector is preferably designed such that the recombinase is either expressed only or substantially only in plastids or is targeted specifically or substantially specifically to plastids.

The promoter in the Recombinase Vector must be one that is operable in the plant cell which is to be transformed. The promoter might, for example, be one derived from a plant or bacterial gene. Preferably, the promoter is plant-specific or plastid-specific. Most preferably, the promoter is an inducible promoter such as XVE (Zuo J, Niu QW, Chua NH. (2000) Technical advance: An estrogen receptor-based transactivator XVE mediates highly inducible gene expression in transgenic plants. Plant J. 24, 265-273.).

In the context of the present invention, the term "plant-specific" means plant-specific or substantially plant-specific. Similarly, the term "plastid-specific" means specific or substantially specific to plastids.

The promoter may or may not be an inducible promoter. If the Recombinase Vector is introduced to the plant cell before or during selection (step (ii)), it is preferable that the promoter is inducible. Examples of inducible promoters which are capable of operating in plants include light inducible promoters, metal inducible promoters, heat-shock promoters and other environmentally-inducible promoters.

Preferably, the promoter is an inducible promoter, for example an XVE promoter (Zuo J, Niu QW, Chua NH. (2000) Technical advance: An estrogen receptor-based transactivator XVE mediates highly inducible gene expression in transgenic plants. Plant J. 24, 265-273.) or a lac promoter.

In one embodiment of the invention, the Recombinase Vector comprises a promoter, operably linked to a nucleotide sequence encoding a plastid-targeting transit peptide and a recombinase.

Upon expression, a polypeptide product will be produced comprising a plastid-targeting transit peptide operably linked to a recombinase polypeptide. In this embodiment, the promoter may or may not be plastid-specific.

In the context of the present invention, the term "plastid-targeting transit peptide" means a peptide sequence which is capable of targeting the recombinase polypeptide to a plastid in a specific or substantially specific manner. Upon expression, the recombinase polypeptide will be produced and specifically imported into plastids by means of the plastid-targeting peptide.

Examples of plastid-targeting transit peptides include plastid-targeting transit peptides from plastid-targeted proteins.

Preferably, the plastid-targeting transit peptide is one which is capable of targeting the recombinase polypeptide to a chloroplast.

Most preferably, the plastid-targeting transit peptide is a plastid-targeting transit peptide from a stromal plastid targeted protein.

Specific examples of plastid-targeting transit peptides include: Transit peptide from AtABC1 (Simon Geir Møller, Tim Kunkel and Nam-Hai Chua. (2001) "A plastidic ABC protein involved in intercompartmental communication of light signaling", Genes and Dev. 15, 90-103.); from AtMinE1 (Jodi Maple, Nam-Hai Chua and Simon Geir Møller (2002) "The topological specificity factor AtMinE1 is required for correct plastid division site placement in Arabidopsis", Plant J. 31, 269-277); and from GIANT CHLOROPLAST 1 (Jodi Maple, Makoto T. Fujiwara, Nobutaka Kitahata, Tracey Lawson, Neil Baker, Shigeo Yoshida and Simon Geir Møller (2004) "GIANT CHLOROPLAST 1 is essential for correct plastid division in Arabidopsis". Current Biology. 14, 776-781)).

Most preferably, the Recombinase Vector comprises an XVE promoter, operably linked to a nucleotide sequence encoding a plastid-targeting transit peptide and CRE recombinase.

The Recombinase Vector may also comprise other elements, for example, the nptII gene (kanamycin resistance) to allow for selection of transformed cells.

Thus in some embodiments of the invention, step (iii) comprises:
(iii) transforming the plant cell with a Recombinase Vector comprising a promoter, a nucleotide sequence encoding a plastid-targeting transit peptide and a recombinase, wherein the recombinase is one which recognises the first and second site-specific recombination elements.

Particularly preferably, step (iii) of the invention comprises:
(iii) transforming the plant cell with a Recombinase Vector comprising a promoter, a nucleotide sequence encoding a plastid-targeting transit peptide and a recombinase, wherein the promoter is capable of driving the expression of the nucleotide sequence encoding the plastid-targeting transit peptide and the recombinase in the plant cell, and wherein, upon expression in the plant cell, the recombinase polypeptide is targeted by the transit peptide to the plastid. More preferably, the promoter is an inducible promoter.

In other embodiments of the invention, the genetic construct additionally comprises the Recombinase Vector as defined herein.

The person skilled in the art will be aware of numerous methods for transforming plant cells with nucleic acid vectors. These include direct DNA uptake into protoplasts, PEG-mediated uptake to protoplasts, microparticle bombardment, electroporation, heat-shock, micro-injection of DNA, microparticle bombardment of tissue explants or cells, vacuum-infiltration of plant tissues, and T-DNA mediated transformation of plant tissues by *Agrobacterium*, and plant (preferably tobacco) liquid cultures.

For transformation of the plant cell containing the selected plastid, any such suitable method may be used.

In some embodiments of the invention, the plant cells to be transformed are guard cells, i.e. stomatal guard cells. Such cells have been shown to be totipotent and therefore regeneration should be more efficient. Guard cells may be used as epidermal strips or as isolated guard cell protoplasts. (Hall et al. 1996. 112 889-892, Plant Physiology; Hall et al. 1996, 14. 1133-1138, Nature Biotechnology).

For targeting the genetic construct to plastids, biolistic transformation is preferred. This involves shooting nucleic acid vector-coated gold particles (micro-projectiles) into plastids of plant tissues, followed by selection of the transformed plastids and plant regeneration. Preferably, the plant tissue is a plant leaf, although callus, as for rice transformation, may also be used.

The method of the invention preferably also comprises the additional step of inducing the expression of the recombinase in the plant cell.

This step will take place after the Recombinase Vector/expressible construct and Transformation Cassette are both present in the plant cell. Preferably, this step will take place after selection of the plant cells on media lacking auxin.

The expression of the recombinase may be induced by applying an inducing agent which results in the activation of the promoter which is present in the Recombinase Vector or endogenous promoter or expressible construct. The recombinase polypeptide is expressed and it then binds to the first and second site-specific recombination elements in the Excision Cassette, leading to the excision of that Cassette. (As will be understood by the person skilled in the art, one of the site-specific recombination elements and some adjacent sequence may be left in the plastid genome).

Once the nucleotide sequences encoding the plant-auxin biosynthetic polypeptides have been removed from the plastid genome, the promoter which is present in the Transformation Cassette will then be able to direct expression of the downstream transgene(s), thus producing the polypeptides(s) of interest.

The invention also provides a method for making a transgene product, comprising the method for producing a transformed plant cell, as claimed in any one of claims 1 to 5, and additionally comprising purifying the transgene product from the plastids.

One embodiment of the invention includes a method for producing a transformed plant cell, the method comprising the step:
(i) transforming the plant cell with a genetic construct,
   wherein the genetic construct comprises first and second homologous recombination elements flanking a Transformation Cassette,
   wherein the first and second homologous recombination elements are capable of directing the integration of the Transformation Cassette into the genome of at least one plastid which is present in the plant cell,
   wherein the Transformation Cassette comprises:
   (a) a first promoter which is operable in said plant cell,
   (b) an Excision Cassette,
   (c) one or more transgenes,
   (d) a first terminator element,
   wherein the Excision Cassette comprises:
   (b1) a first site-specific recombination element,
   (b2) an optional second promoter
   (b3) one or more nucleotide sequences encoding one or more plant-auxin biosynthetic polypeptides, wherein the plant-auxin biosynthetic polypeptides are selected from iaaH (indoleacetamide hydrolase) and iaaM (tryptophan mono-oxygenase),
   (b4) a second terminator element,
   (b5) a second site-specific recombination element,
   wherein the first and second site-specific recombination elements are capable of being recognised by a recombinase, and wherein the first and second site-specific recombination elements are lox elements and the recombinase is Cre.

The invention further provides a plant plastid comprising a Transformation Cassette of the invention, a plant plastid comprising a Recombinase Vector of the invention, and a plant plastid comprising a Transformation Cassette and a Recombinase Vector of the invention.

Additionally, the invention provides a plant cell obtainable or obtained using a method of the invention.

### BRIEF DESCRIPTION OF THE FIGURES

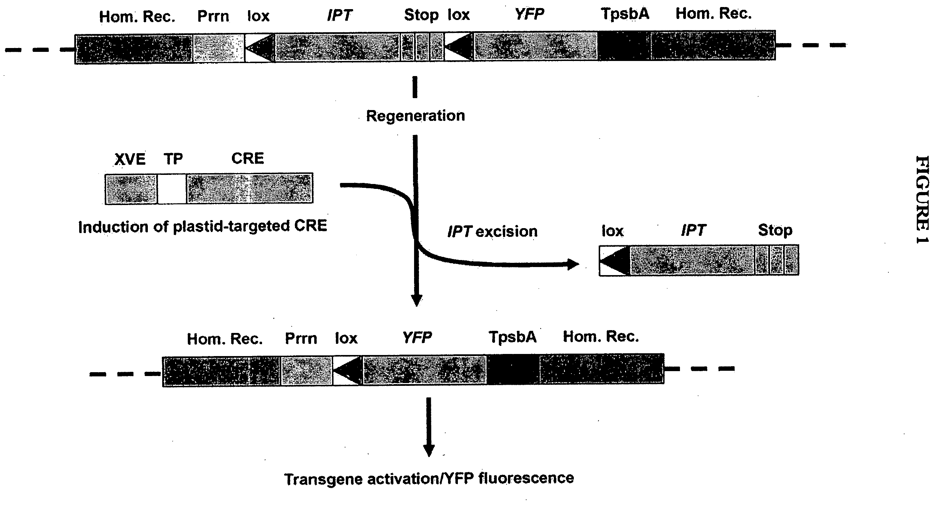

**Figure 1****: Schematic diagram showing the overall principle of the IPT gene excision and YFP gene activation.**
   pPTI001-YFP containing the IPT gene sandwiched between two lox sites which allows for CRE-mediated IPT excision after regeneration. The removal of the IPT gene results in simultaneous transgene (YFP) activation.

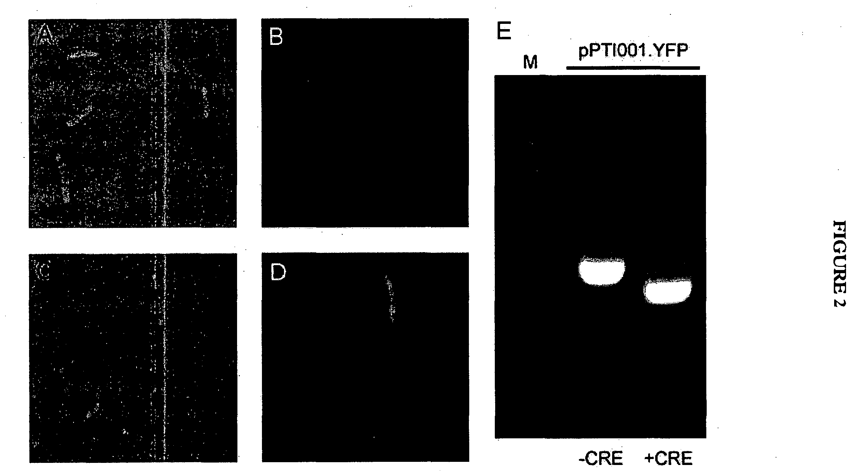

**Figure 2****: CRE induced excision of the IPT cassette in pPTI001-YFP.**
   Bright field (A) and fluorescence (B) images of *E. coli* DH5α cells containing the pPTI001-YFP vector. Bright field (C) and fluorescence (D) images of *E*. *coli* DH5α cells containing both the pPTI001-YFP and pER10-TP.CRE vectors (Figure 1). CRE induced excision of the IPT cassette in pPTI001-YFP results in constitutive expression of YFP from the Prrn promoter. (E) PCR confirmation of CRE induced excision of the IPT cassettes in the pPTI001-YFP vector using primers spanning the TrbcL and TpsbA terminators (Figure 3). M: molecular weight marker.
**Figure 3****: Schematic diagram of the (A) pPTI001 and (B) pPTI001-YFP vectors.**
   **HOM1:** Homologous recombination sequence (nt 104091-105380, accession Z00044 *Nicotiana tabacum* chloroplast genome DNA); **Prrn:** Plastidic ribosomal RNA (*rrn*) operon promoter (nt 59034-59303, accession Z00044 *Nicotiana tabacum* chloroplast genome DNA); **IPT:** isopentenyltranferase gene from *Agrobacterium tumefaciens;* **TrbcL:** *Ribulose-1,5-Bisphosphate Carboxylase*/*Oxygenase polyA* addition sequence (nt 102539-102685, accession Z00044 *Nicotiana tabacum* chloroplast genome DNA); **YFP:** Yellow fluorescence protein; **TpsbA:** *psbA* polyA addition sequence; **HOM2:** Homologous recombination sequence (nt 105381-106370, accession Z00044 *Nicotiana tabacum* chloroplast genome DNA).

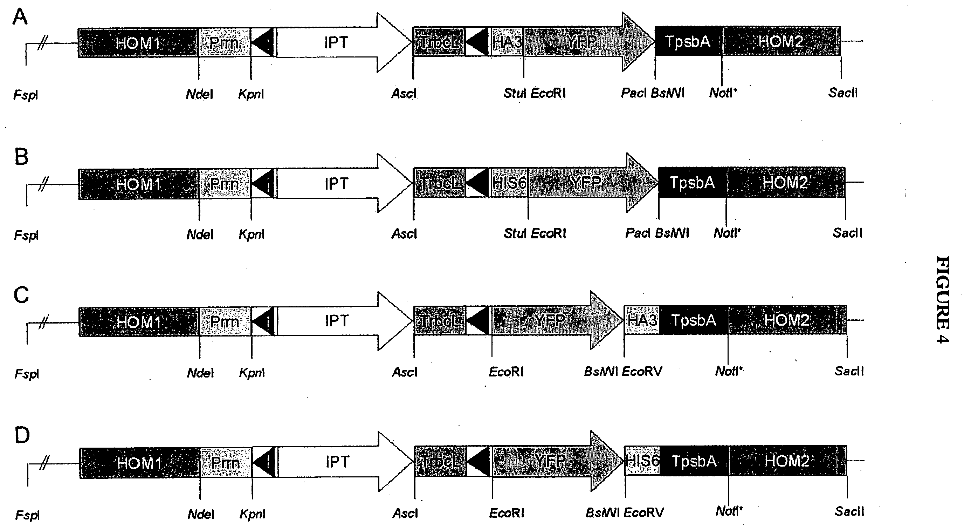

**Figure 4****: Schematic diagram of the modified pPTI001-YFP vector containing protein purification tags (pPTI001 vector series).**
   (A) pPTI001a-YFP, pPTI001-YFP containing an N-terminal influenza hemagglutinin-HA-epitope tag (HA3).
   (B) pPTI001b-YFP, pPTI001-YFP containing six N-terminal histidine amino acids (HIS6).
   (C) pPTI001c-YFP, pPTI001-YFP containing a C-terminal influenza hemagglutinin-HA-epitope tag (HA3).
   (D) pPTI001d-YFP, pPTI001-YFP containing six C-terminal histidine amino acids (HIS6).

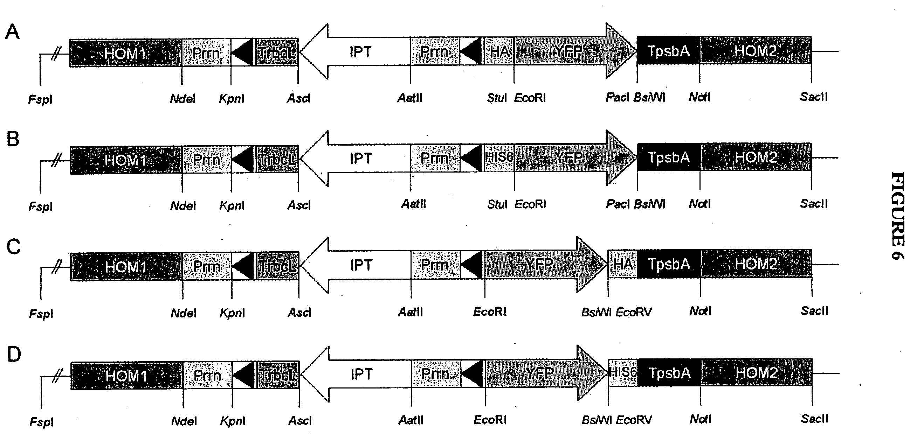

   To allow for the use of EcoRV as a cloning site downstream from YFP in (C) pPTI001c-YFP and (D) pPTI001d-YFP the IPT DNA sequence was modified changing adenine to guanidine at nucleotide position 519 (+1 taken as adenine in the start codon) thereby removing an endogenous EcoRV site.
**Figure 5****: Schematic diagram of the (A) pPTI002 and (B) pPTI002-YFP vectors.**
   **HOM1:** Homologous recombination sequence (nt 104091-105380, accession Z00044 *Nicotiana tabacum* chloroplast genome DNA); **Prrn:** Plastidic ribosomal RNA (*rrn*) operon promoter (nt 59034-59303, accession Z00044 *Nicotiana tabacum* chloroplast genome DNA); **IPT:** isopentenyltranferase gene from *Agrobacterium tumefaciens;* **TrbcL:** *Ribulose-1,5-Bisphosphate Carboxylase*/*Oxygenase polyA* addition sequence (nt 102539-102685, accession Z00044 *Nicotiana tabacum* chloroplast genome DNA); **YFP:** Yellow fluorescence protein; **TpsbA:** *psbA* polyA addition sequence; **HOM2:** Homologous recombination sequence (nt 105381-106370, accession Z00044 *Nicotiana tabacum* chloroplast genome DNA).
   The construction of the vector series pPTI002 (see also Figure 6) is to ensure that there is no leaky expression from the Prrn promoter to the transgene in question (in this case YFP) prior to CRE-mediated excision. Although this does not seem to present a real problem, as shown in Figure 2, we have placed the IPT gene and the YFP gene in opposite orientations.
**Figure 6****: Schematic diagram of the modified pPTI002-YFP vector containing protein purification tags (pPTI002 vector series).**
   (A) pPTI002a-YFP, pPTI002-YFP containing an N-terminal influenza hemagglutinin-HA-epitope tag (HA).
   (B) pPTI002b-YFP, pPTI002-YFP containing six N-terminal histidine amino acids (HIS6).
   (C) pPTI002c-YFP, pPTI002-YFP containing a C-terminal influenza hemagglutinin-HA-epitope tag (HA).
   (D) pPTI002d-YFP, pPTI002-YFP containing six C-terminal histidine amino acids (HIS6).

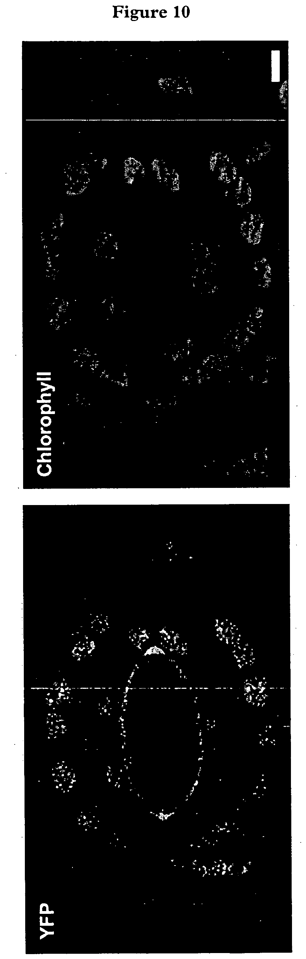

   To allow for the use of EcoRV as a cloning site downstream from YFP in (C) pPTI002c-YFP and (D) pPTI002d-YFP, the IPT DNA sequence was modified changing adenine to guanidine at nucleotide position 519 (+1 taken as adenine in the start codon) thereby removing an endogenous EcoRV site.

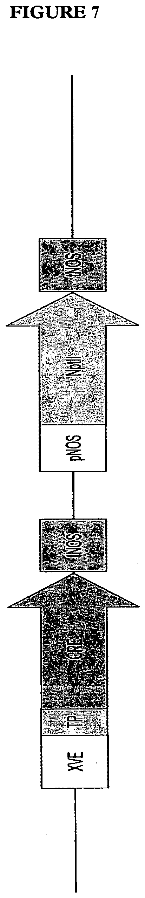

**Figure 7****: Schematic diagram of pER10/TPCRE.**
   XVE acts as the inducible promoter that drives TP-CRE (Transit peptide fused to CRE) expression. Selection of this transgene is by Kanamycin resistance conferred by the *nptII* gene.

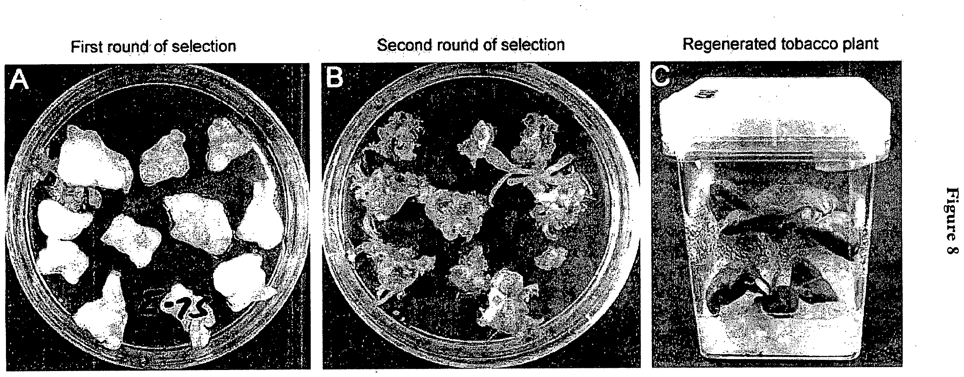

**Figure 8****: Selection and regeneration of positive transplastomic plants using the IPT selectable marker.**
   A) First round of selection after plastid transformation showing the presence of new shoots in the absence of cytokinin.
   B) Second round of selection from the primary transformant shown in A.
   C) Regenerated transplastomic tobacco plant used for CRE infiltration and GFP activation (Figure 10) .

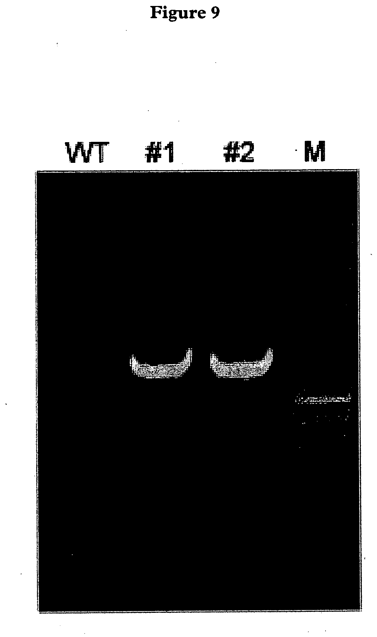

**Figure 9****: Confirmation of transgene insertion into the tobacco plastid genome.** Insertion of pPTI001/YFP into the tobacco chloroplast genome at the homologous recombination sites was confirmed using a primer in the flanking region of the tobacco chloroplast genome and a primer that anneals to TrbcL terminator within the cassette.
   Lanes shown: WT, wild-type; #1, regenerant 1; #2 regenerant 2; M, marker.
**Figure 10****: Induction of YFP expression in tobacco leaf cells.**
   Regenerants transplastomic tobacco plant harbouring the pPTI001/YFP transformation cassette analysed by fluorescence microscopy after infiltration and induction of pER10/TP.CRE. Extended focus images of reconstituted YFP fluorophore (YFP) and chlorophyll autofluorescence (Chlorophyll) were captured by epifluorescence microscopy using Volocity II software. Scale bar = 5 µm.

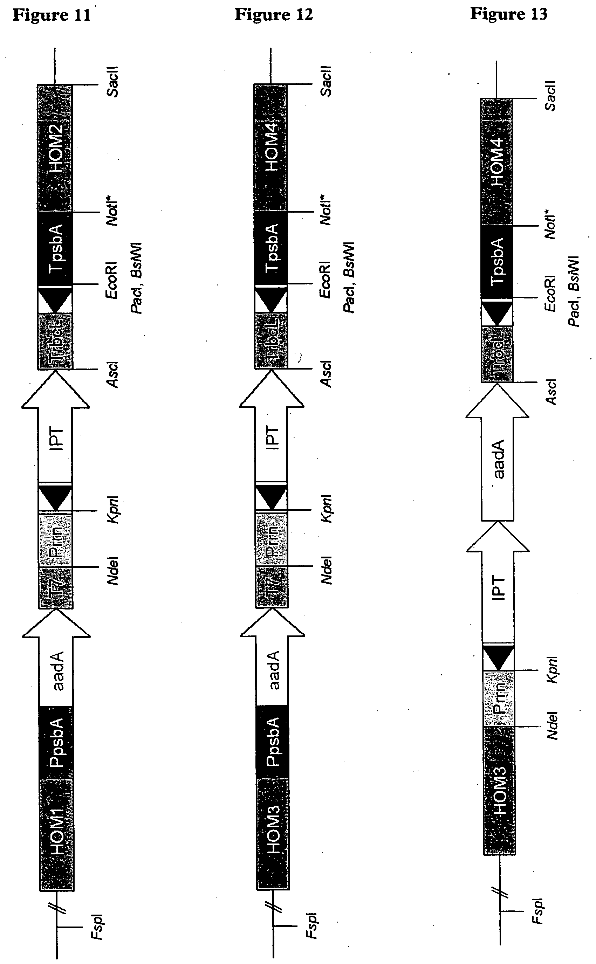

**Figure 11****: Plastid transformation vector pPTI005:**
   **HOM1:** Homologous recombination sequence (nt 104091-105380, accession Z00044 *Nicotiana tabacum* chloroplast genome DNA); **PpsbA:** Plastidic psbA promoter; **aadA:** spectinomycin adenyltransferase gene; **T7:** T7 transcription terminator sequence: **Prrn:** Plastidic ribosomal RNA (*rrn*) operon promoter (nt 59034-59303, accession Z00044 *Nicotiana tabacum* chloroplast genome DNA); **IPT:** isopentenyltranferase gene from *Agrobacterium tumefaciens*; **TrbcL:** *Ribulose-1,5-Bisphosphate Carboxylase*/*Oxygenase polyA* addition sequence (nt 102539-102685, accession Z00044 *Nicotiana-tabacum* chloroplast genome DNA); **TpsbA:** *psbA* polyA addition sequence; **HOM2:** Homologous recombination sequence (nt 105381-106370, accession Z00044 *Nicotiana tabacum* chloroplast genome DNA).
**Figure 12****: Plastid transformation vector pPTI007:**
   **HOM3:** Homologous recombination sequence (nt 102925-101857, accession Z00044 *Nicotiana tabacum* chloroplast genome DNA); **PpsbA:** Plastidic psbA promoter; **aadA:** spectinomycin adenyltransferase gene; **T7:** T7 transcription terminator sequence: **Prrn:** Plastidic ribosomal RNA (*rrn*) operon promoter (nt 59034-59303, accession Z00044 *Nicotiana tabacum* chloroplast genome DNA); **IPT:** isopentenyltranferase gene from *Agrobacterium tumefaciens;* **TrbcL:** *Ribulose-1,5-Bisphosphate Carboxylase*/*Oxygenase polyA* addition sequence (nt 102539-102685, accession Z00044 *Nicotiana tabacum* chloroplast genome DNA); **TpsbA:** *psbA* polyA addition sequence; **HOM4:** Homologous recombination sequence (nt 100933-100130, accession Z00044 *Nicotiana tabacum* chloroplast genome DNA).
**Figure 13****: Plastid transformation vector pPTI008:**
   **HOM3:** Homologous recombination sequence (nt 102925-101857, accession Z00044 *Nicotiana tabacum* chloroplast genome DNA); **Prrn:** Plastidic ribosomal RNA (*rrn*) operon promoter (nt 59034-59303, accession Z00044 *Nicotiana tabacum* chloroplast genome DNA); **IPT:** isopentenyltranferase gene from *Agrobacterium tumefaciens*; **aadA:** spectinomycin adenyltransferase gene; **TrbcL:** *Ribulose-1,5-Bisphosphate Carboxylase*/*Oxygenase polyA* addition sequence (nt 102539-102685, accession Z00044 *Nicotiana tabacum* chloroplast genome DNA); **TpsbA:** *psbA* polyA addition sequence; HOM4: Homologous recombination sequence (nt 100933-100130, accession Z00044 *Nicotiana tabacum* chloroplast genome DNA). The *IPT.aadA* cassette is constructed as an operon. Both of the *IPT* and *aadA* genes are preceded by a shine delgardo sequence and a six base pair spacer.

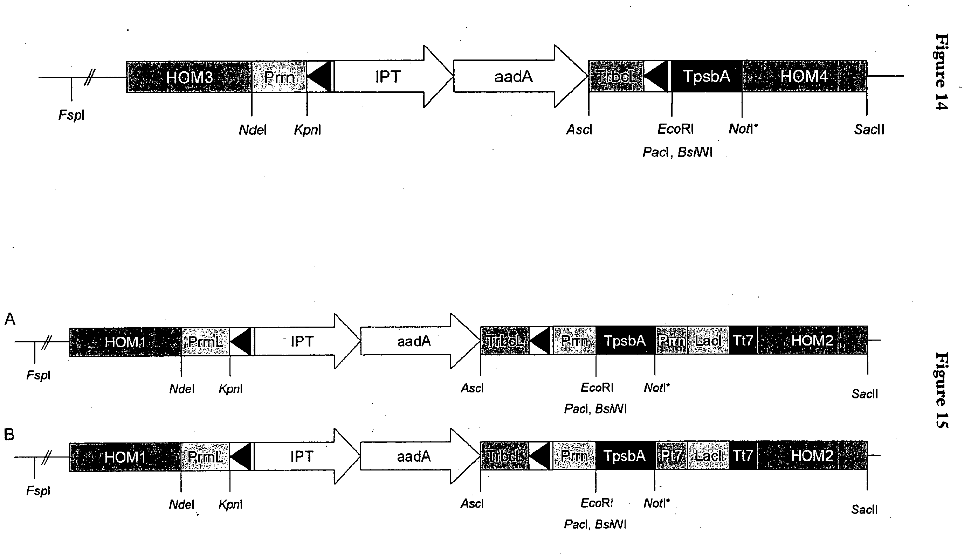

**Figure 14****: Plastid transformation vector pPTI009:**
   **HOM3:** Homologous recombination sequence (nt 102925-101857, accession Z00044 *Nicotiana tabacum* chloroplast genome DNA); **Prrn:** Plastidic ribosomal RNA (*rrn*) operon promoter (nt 59034-59303, accession Z00044 *Nicotiana tabacum* chloroplast genome DNA); **IPT:** isopentenyltranferase gene from *Agrobacterium tumefaciens*; **aadA:** spectinomycin adenyltransferase gene; **TrbcL:** *Ribulose-1,5-Bisphosphate Carboxylase*/*Oxygenase polyA* addition sequence (nt 102539-102685, accession Z00044 *Nicotiana tabacum* chloroplast genome DNA); **TpsbA:** *psbA* polyA addition sequence; **HOM4:** Homologous recombination sequence (nt 100933-100130, accession Z00044 *Nicotiana tabacum* chloroplast genome DNA). The *IPT.aadA* cassette is expressed as a fusion protein. The *IPT* a gene is preceded by a shine delgardo sequence and a six base pair spacer and the two open reading frames are separated by an eight glycine linker sequence.
**Figure 15****: Plastid transformation vectors pinPTI.**
   Each pPTI vector contains a constitutively expressed *LacI* gene. pPTI003 is shown as an example. Modified Prrn promotors (PrrnL) will be inserted upstream of the IPT.aadA cassette, allowing inducible, controlled expression of the IPT.aadA cassette. (A) pind1PTI, pPTI containing the LacI open reading frame under the control of the Prrn promoter sequence and modified PrrnL promoter upstream of the IPT.aadA cassette. (B) pind2PTI, pPTI containing the *LacI* open reading frame under the control of the T7 promoter sequence and modified PrrnL promoter upstream of the IPT.aadA cassette. **HOM1:** Homologous recombination sequence (nt 104091-105380, accession Z00044 *Nicotiana tabacum* chloroplast genome DNA); **Prrn:** Plastidic ribosomal RNA (*rrn*) operon promoter (nt 59034-59303, accession Z00044 *Nicotiana tabacum* chloroplast genome DNA); **IPT:** isopentenyltranferase gene from *Agrobacterium tumefaciens;* **aadA:** spectinomycin adenyltransferase gene; **TrbcL:** *Ribulose-1,5-Bisphosphate Carboxylase*/*Oxygenase polyA* addition sequence (nt 102539-102685, accession Z00044 *Nicotiana tabacum* chloroplast genome DNA); **TpsbA:** *psbA* polyA addition sequence; HOM2: Homologous recombination sequence (nt 105381-106370, accession Z00044 *Nicotiana tabacum* chloroplast genome DNA). The *IPT.aadA* cassette is constructed as an operon. Both of the *IPT* and *aadA* genes are preceded by a shine delgardo sequence and a six base pair spacer.

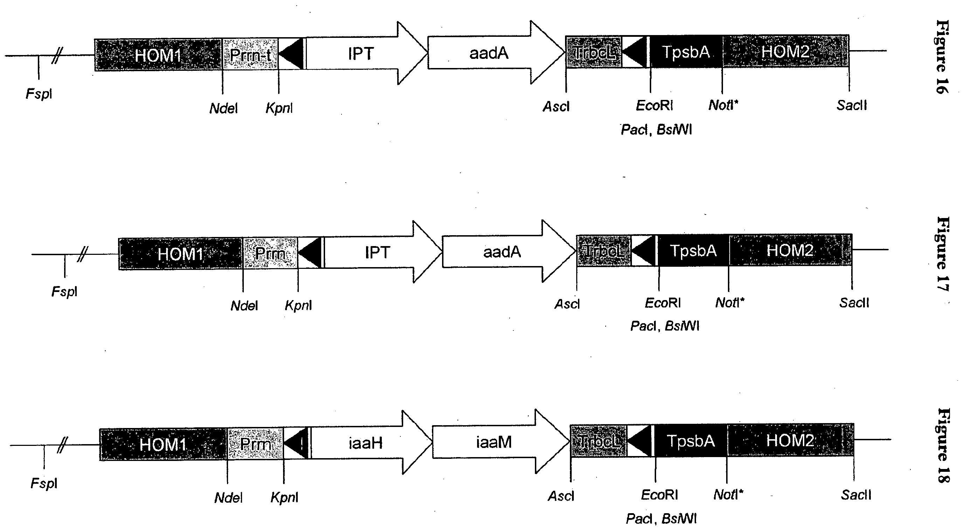

**Figure 16****: Plastid transformation vectors pPTI+**
   Each pPTI vector will be modified to contain a modified Prrn-trbcL (Prrn promoter construct, composed of the Prrn promter and *rbcL* 5' translation control region, to ultimately produce higher levels of expression of the foreign proteins. pPTI003 is shown as an example. **HOM1:** Homologous recombination sequence (nt 104091-105380, accession Z00044 *Nicotiana tabacum* chloroplast genome DNA); **Prrn-t:** Plastidic ribosomal RNA (*rrn*) operon promoter (nt 59034-59303, accession Z00044 *Nicotiana tabacum* chloroplast genome DNA) and *rbcL* 5' translation control region; **IPT:** isopentenyltranferase gene from *Agrobacterium tumefaciens*; **aadA:** spectinomycin adenyltransferase gene; **TrbcL:** *Ribulose-1,5-Bisphosphate Carboxylase*/*Oxygenase polyA* addition sequence (nt 102539-102685, accession Z00044 *Nicotiana tabacum* chloroplast genome DNA); **TpsbA:** *psbA* polyA addition sequence; **HOM2:** Homologous recombination sequence (nt 105381-106370, accession Z00044 *Nicotiana tabacum* chloroplast genome DNA). The *IPT.aadA* cassette is constructed as an operon. Both of the *IPT* and *aadA* genes are preceded by a shine delgardo sequence and a six base pair spacer.
**Figure 17****: pPTI003**
   **HOM1:** Homologous recombination sequence (nt 104091-105380, accession Z00044 *Nicotiana tabacum* chloroplast genome DNA); **Prrn:** Plastidic ribosomal RNA (rrn) operon promoter (nt 59034-59303, accession Z00044 *Nicotiana tabacum* chloroplast genome DNA); **IPT:** isopentenyltranferase gene from *Agrobacterium tumefaciens;* **aadA:** spectinomycin adenyltransferase gene; **TrbcL:** Ribulose-1,5-Bisphosphate Carboxylase/Oxygenase polyA addition sequence (nt 102539-102685, accession Z00044 *Nicotiana tabacum* chloroplast genome DNA); **TpsbA:** psbA polyA addition sequence; **HOM2:** Homologous recombination sequence (nt 105381-106370, accession Z00044 *Nicotiana tabacum* chloroplast genome DNA). The IPT.aadA cassette is constructed as an operon. Both of the IPTand aadA genes are preceded by a shine delgardo sequence and a six base pair spacer.
      **pPTI004**
   **HOM1:** Homologous recombination sequence (nt 104091-105380, accession Z00044 *Nicotiana tabacum* chloroplast genome DNA); **Prrn:** Plastidic ribosomal RNA (rrn) operon promoter (nt 59034-59303, accession Z00044 *Nicotiana tabacum* chloroplast genome DNA); **IPT:** isopentenyltranferase gene from *Agrobacterium tumefaciens*; **aadA:** spectinomycin adenyltransferase gene; **TrbcL:** Ribulose-1,5-Bisphosphate Carboxylase/Oxygenase polyA addition sequence (nt 102539-102685, accession Z00044 *Nicotiana tabacum* chloroplast genome DNA); **TpsbA:** psbA polyA addition sequence; **HOM2:** Homologous recombination sequence (nt 105381-106370, accession Z00044 *Nicotiana tabacum* chloroplast genome DNA). The IPT.aadA cassette is expressed as a fusion protein. The IPT a gene is preceded by a shine delgardo sequence and a six base pair spacer and the two open reading frames are separated by an eight glycine linker sequence.
**Figure 18****: Plastid transformation vector pPTA001**
   **HOM1:** Homologous recombination sequence (nt 104091-105380, accession Z00044 *Nicotiana tabacum* chloroplast genome DNA); **Prrn:** Plastidic ribosomal RNA (rrn) operon promoter (nt 59034-59303, accession Z00044 *Nicotiana tabacum* chloroplast genome DNA); **iaaH:** indoleacetamide hydrolase from *Agrobacterium tumefaciens;* **iaaM:** tryptophan monooxygenase from *Agrobacterium tumefaciens;* **TrbcL:** Ribulose-1,5-Bisphosphate Carboxylase/Oxygenase polyA addition sequence (nt 102539-102685, accession Z00044 *Nicotiana tabacum* chloroplast genome DNA); **TpsbA:** psbA polyA addition sequence; **HOM2:** Homologous recombination sequence (nt 105381-106370, accession Z00044 *Nicotiana tabacum* choloroplast genome DNA). The iaaH.iaaM cassette is constructed as an operon. Both of the iaaH and iaaM genes are preceded by a shine delgardo sequence and a six base pair spacer.

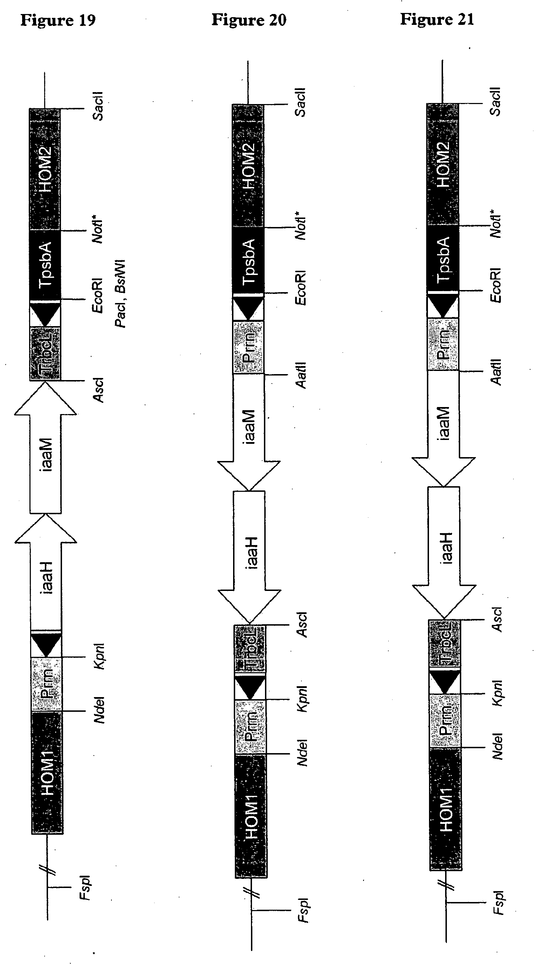

**Figure 19****: Plastid transformation vector pPTA002**
   **HOM1:** Homologous recombination sequence (nt 104091-105380, accession Z00044 *Nicotiana tabacum* chloroplast genome DNA); **Prrn:** Plastidic ribosomal RNA (rrn) operon promoter (nt 59034-59303, accession Z00044 *Nicotiana tabacum* chloroplast genome DNA); **iaaH:** indoleacetamide hydrolase from *Agrobacterium tumefaciens;* **iaaM:** tryptophan monooxygenase from *Agrobacterium tumefaciens;* **TrbcL:** Ribulose-1,5-Bisphosphate Carboxylase/Oxygenase polyA addition sequence (nt 102539-102685, accession Z00044 *Nicotiana tabacum* chloroplast genome DNA); **TpsbA:** psbA polyA addition sequence; **HOM2:** Homologous recombination sequence (nt 105381-106370, accession Z00044 *Nicotiana tabacum* chloroplast genome DNA). The iaaH.iaaM cassette is expressed as a fusion protein. The iaaH a gene is preceded by a shine delgardo sequence and a six base pair spacer and the two open reading frames are separated by an eight glycine linker sequence.
**Figure 20****: Plastid transformation vector pPTA003**
   **HOM1:** Homologous recombination sequence (nt 104091-105380, accession Z00044 *Nicotiana tabacum* chloroplast genome DNA); **TrbcL:** Ribulose-1,5-Bisphosphate Carboxylase/Oxygenase polyA addition sequence (nt 102539-102685, accession Z00044 *Nicotiana tabacum* chloroplast genome DNA); **iaaH:** indoleacetamide hydrolase from *Agrobacterium tumefaciens;* **iaaM:** tryptophan monooxygenase from *Agrobacterium tumefaciens;* **Prrn:** Plastidic ribosomal RNA (rrn) operon promoter (nt 59034-59303, accession Z00044 *Nicotiana tabacum* chloroplast genome DNA); **TpsbA:** psbA polyA addition sequence; **HOM2:** Homologous recombination sequence (nt 105381-106370, accession Z00044 *Nicotiana tabacum* chloroplast genome DNA). The iaaH.iaaM cassette is constructed as an operon. Both of the iaaH and iaaM genes are preceded by a shine delgardo sequence and a six base pair spacer.
**Figure 21****: Plastid transformation vector pPTA004**
   **HOM1:** Homologous recombination sequence (nt 104091-105380, accession Z00044 *Nicotiana tabacum* chloroplast genome DNA); **TrbcL:** Ribulose-1,5-Bisphosphate Carboxylase/Oxygenase polyA addition sequence (nt 102539-102685, accession Z00044 *Nicotiana tabacum* chloroplast genome DNA); **iaaH:** indoleacetamide hydrolase from *Agrobacterium tumefaciens;* **iaaM:** tryptophan monooxygenase from *Agrobacterium tumefaciens;* **Prrn:** Plastidic ribosomal RNA (rrn) operon promoter (nt 59034-59303, accession Z00044 *Nicotiana tabacum* chloroplast genome DNA); **TpsbA:** psbA polyA addition sequence; **HOM2:** Homologous recombination sequence (nt 105381-106370, accession Z00044 *Nicotiana tabacum* chloroplast genome DNA). The iaaH.iaaM cassette is expressed as a fusion protein. The iaaH a gene is preceded by a shine delgardo sequence and a six base pair spacer and the two open reading frames are separated by an eight glycine linker sequence.

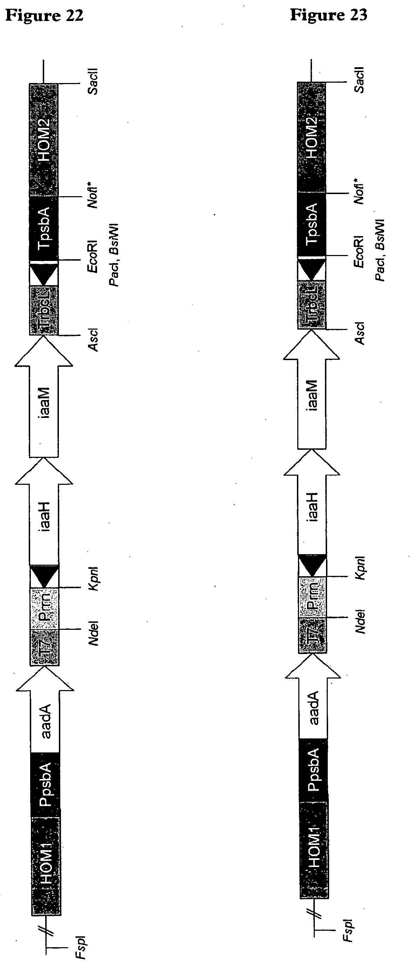

**Figure 22****: Plastid transformation vector pPTA005**
   **HOM1:** Homologous recombination sequence (nt 104091-105380, accession Z00044 *Nicotiana tabacum* chloroplast genome DNA); **PpsbA:** Plastidic psbA promoter; **aadA:** spectinomycin adenyltransferase gene; T7: T7 transcription terminator sequence: **Prrn:** Plastidic ribosomal RNA (rrn) operon promoter (nt 59034-59303, accession Z00044 *Nicotiana tabacum* chloroplast genome DNA); **iaaH:** indoleacetamide hydrolase from *Agrobacterium tumefaciens;* **iaaM:** tryptophan monooxygenase from *Agrobacterium tumefaciens;* **TrbcL:** Ribulose-1,5-Bisphosphate Carboxylase/Oxygenase polyA addition sequence (nt 102539-102685, accession Z00044 *Nicotiana tabacum* chloroplast genome DNA); **TpsbA:** psbA polyA addition sequence; **HOM2:** Homologous recombination sequence (nt 105381-106370, accession Z00044 *Nicotiana tabacum* chloroplast genome DNA). The iaaH.iaaM cassette is constructed as an operon. Both of the iaaH and iaaM genes are preceded by a shine delgardo sequence and a six base pair spacer.
**Figure 23****: Plastid transformation vector pPTA006**
   **HOM1:** Homologous recombination sequence (nt 104091-105380, accession Z00044 *Nicotiana tabacum* chloroplast genome DNA); **PpsbA:** Plastidic psbA promoter; **aadA:** spectinomycin adenyltransferase gene; **T7:** T7 transcription terminator sequence: **Prrn:** Plastidic ribosomal RNA (rrn) operon promoter (nt 59034-59303, accession Z00044 *Nicotiana tabacum* chloroplast genome DNA); **iaaH:** indoleacetamide hydrolase from *Agrobacterium tumefaciens;* **iaaM:** tryptophan monooxygenase from *Agrobacterium tumefaciens;* **TrbcL:** Ribulose-1,5-Bisphosphate Carboxylase/Oxygenase polyA addition sequence (nt 102539-102685, accession Z00044 *Nicotiana tabacum* chloroplast genome DNA); **TpsbA:** psbA polyA addition sequence; **HOM2:** Homologous recombination sequence (nt 105381-106370, accession Z00044 *Nicotiana tabacum* chloroplast genome DNA). The iaaH.iaaM cassette is expressed as a fusion protein. The iaaH a gene is preceded by a shine delgardo sequence and a six base pair spacer and the two open reading frames are separated by an eight glycine linker sequence.

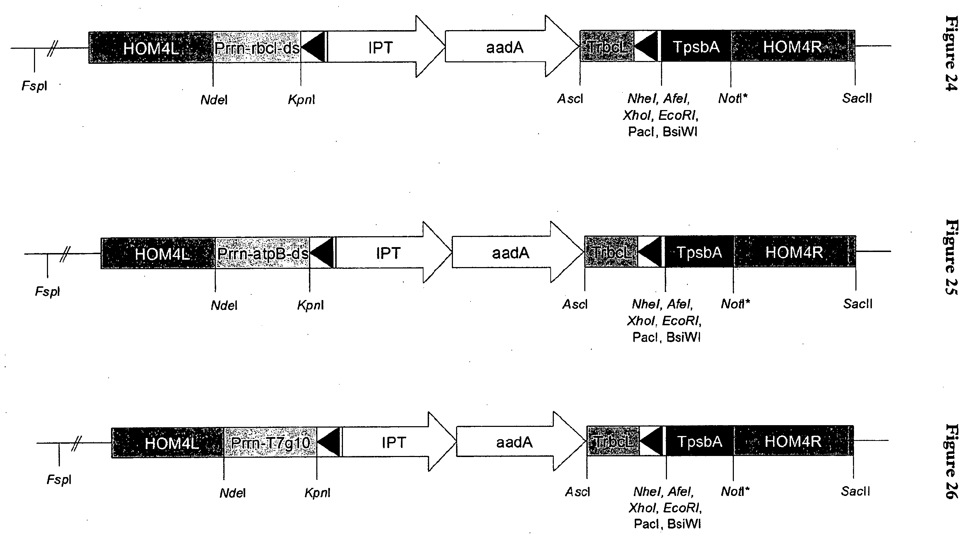

**Figure 24****: Plastid transformation vector pPTS4**
   **HOM4L:** Homologous recombination sequence (nt 104065-102312, accession Z00044 *Nicotiana tabacum* chloroplast genome DNA); **Prrn**-**rbcl-ds:** Plastidic ribosomal RNA (*rrn*) operon promoter (nt 59034-59303, accession Z00044 *Nicotiana tabacum* chloroplast genome DNA), rbcl is the 5'-UTR of the plastid rbcL gene and the downstream sequence (ds) sequence encodes RbcL amino acids 1-14 (Kuroda and Maliga (2001). Sequences downstream of the translation initiation codon are important determinants of translation efficiency in chloroplasts. Plant Physiol. volume 125(1) Page 431, Figure 1); **IPT:** isopentenyltranferase gene from *Agrobacterium tumefaciens*; **aadA:** spectinomycin adenyltransferase gene; **TrbcL:** Ribulose-1,5-Bisphosphate Carboxylase/Oxygenase polyA addition sequence (nt 102539-102685, accession Z00044 *Nicotiana tabacum* chloroplast genome DNA); **TpsbA:** psbA polyA addition sequence; **HOM4R:** Homologous recombination sequence (nt 105381-106370, accession Z00044 *Nicotiana tabacum* chloroplast genome DNA).
**Figure 25****: Plastid transformation vector pPTS5**
   **HOM4L:** Homologous recombination sequence (nt 104065-102312, accession Z00044 *Nicotiana tabacum* chloroplast genome DNA); **Prrn**-**atpB-ds:** Plastidic ribosomal RNA (*rrn*) operon promoter (nt 59034-59303, accession Z00044 *Nicotiana tabacum* chloroplast genome DNA), atpb is the 5'-UTR of the plastid atpb gene and the downstream sequence (ds) sequence encodes AtpB amino acids 1-14 (Kuroda and Maliga (2001). Sequences downstream of the translation initiation codon are important determinants of translation efficiency in chloroplasts. Plant Physiol. volume 125(1) Page 431, Figure 1); **IPT:** isopentenyltranferase gene from *Agrobacterium tumefaciens*; **aadA:** spectinomycin adenyltransferase gene; **TrbcL:** Ribulose-1,5-Bisphosphate Carboxylase/Oxygenase polyA addition sequence (nt 102539-102685, accession Z00044 *Nicotiana tabacum* chloroplast genome DNA); **TpsbA:** psbA polyA addition sequence; **HOM4R:** Homologous recombination sequence (nt 105381-106370, accession Z00044 *Nicotiana tabacum* chloroplast genome DNA).
**Figure 26****: Plastid transformation vector pPTS6**
   **HOM4L:** Homologous recombination sequence (nt 104065-102312, accession Z00044 *Nicotiana tabacum* chloroplast genome DNA); **Prrn-T7g10:** Plastidic ribosomal RNA (*rrn*) operon promoter (nt 59034-59303, accession Z00044 *Nicotiana tabacum* chloroplast genome DNA), T7g10 is the 5'-UTR of the *E. coli* phage T7 gene 10 (Kuroda and Maliga (2001) Complementarity of the 16S rRNA penultimate stem with sequences downstream of the AUG destabilizes the plastid mRNAs. Nucleic Acids Res. volume 29(4) page 972, Figure 1); **IPT:** isopentenyltranferase gene from *Agrobacterium tumefaciens*; **aadA:** spectinomycin adenyltransferase gene; **TrbcL:** Ribulose-1,5-Bisphosphate Carboxylase/Oxygenase polyA addition sequence (nt 102539-102685, accession Z00044 *Nicotiana tabacum* chloroplast genome DNA); **TpsbA:** psbA polyA addition sequence; **HOM4R:** Homologous recombination sequence (nt 105381-106370, accession Z00044 *Nicotiana tabacum* chloroplast genome DNA).

The present invention is further described in the following Examples, in which parts and percentages are by weight and degrees are Celsius, unless otherwise stated. It should be understood that these Examples, are given by way of illustration only.

### EXAMPLES

### Example 1

The plastid transformation vectors pPTI001 and pPTI002 were constructed as detailed in Figure 3 and Figure 5 using a 1289 bp homologous recombination sequence (104091-105380 nt) and a 989 bp homologous recombination sequence (105381-106370 nt) from the chloroplast genome from *Nicotiana tabacum* (Shinozaki K, Ohme M, Tanaka M, Wakasugi T, Hayashida N, Matsubayashi T, Zaita N, Chunwongse J, Obokata J, Yamaguchi-Shinozaki K, Ohto C, Torazawa K, Meng BY, Sugita M, Deno H, Kamogashira T, Yamada K, Kusuda J, Takaiwa F, Kato A, Tohdoh N, Shimada H, Sugiura M. (1986) The complete nucleotide sequence of the tobacco chloroplast genome: its gene organization and expression. EMBO J. 5, 2043-2049.) on either side of the gene cassette. Shorter homologous recombination sequences from the *Nicotiana tabacum* chloroplast DNA (157 and 143 bp in length) have also been used showing a 3-fold increase in plastid transformation efficiencies.

For proof-of-principle purposes, the Yellow Fluorescent Protein (YFP) reporter gene was cloned into pPTI001 and pPTI002 to generate pPTI001-YFP (Figure 3) and pPTI002-YFP (Figure 5). These vectors where then bombarded into tobacco leaves as detailed in Appendix 1. For transformation experiments, leaves from plants growing in the greenhouse were used instead of in Magenta Box as this increases the transformation efficiency. Leaves were sterilized in 10% Super Bleach Sterilizer (Coventry) for ten minutes and washed in sterilized water three times. The leaves were then cut to fit the plates for bombardment. Following these strategies, the transformation efficiency increased nearly ten times.

Because the pPTI001 and pPTI002 vector series also functions in bacteria, we tested whether the CRE-mediated excision of the IPT gene was functional ultimately leading to YFP gene activation and YFP fluorescence in *E. coli* cells. A schematic diagram showing the principal of the system is shown in Figure 1. Chemically competent *E. coli* DH5α cells were produced and transformed with the vector pER10-TP.CRE, which constitutively expresses the TP.CRE fusion protein, and selected on LB media containing spectinomycin. Subsequently chemically competent E. coli DH5α cells containing the pER10-TP.CRE vector were produced and transformed with pPTI001-YFP. Cells containing both vectors were selected for on LB media containing spectinomycin and chloramphenicol. Single colonies were inoculated into LB media containing spectinomycin and chloramphenicol and grown to an OD600 of 0.4 before analysis for YFP expression on a Nikon TE-2000U inverted fluorescence microscope equipped with filters for YFP (exciter HQ500/20, emitter S535/30) fluorescence and a Hamamatsu Orca ER 1394 cooled CCD camera. Images were captured using Openlab software (Improvision). PCR verification of IPT excision was carried out on plasmid DNA prepared from the *E. coli* cells and the primers TrbcL-F and TpsbA-R which span the IPT cassette region. Figure 2 shows no excision of the IPT gene prior to addition of CRE (Figures 2A and 2E) whilst after CRE addition the IPT gene is removed at the molecular level (Figure 2E) leading to YFP expression (Figure 2D).

### Example 2

### Expression of proteins with toxic effects on plant development

The expression of high levels of foreign protein in plants can lead to detrimental effects on plant development because of toxic effects. The described system overcomes this by combining insertion of the transgene(s) into the plastid genome where it remains dormant until the IPT selectable marker gene is removed by CRE/lox mediated recombination.

The transgene encoding the "plant-toxic" protein is inserted into one of the pPTI001 or pPTI002 vectors (Figure 3 and 5) between the TrbcL and the TspbA polyA addition sequences in the pPTI001 vector series or between the Prrn promoter and the TspbA polyA addition sequence in the pPTI002 vector series and the construct transformed into plastids using the protocol shown in Appendix 1 followed by cytokinin-mediated selection and regeneration. Once regenerated, the IPT gene is removed by CRE-mediated recombination and the toxic transgene is activated leading to minimal adverse effects on initial plant regeneration. Once expressed, the recombinant protein can be purified using one of the affinity tags present in either the pPTI001 or pPTI002 vector series shown in Figures 4 and 6.

### Example 3

### Expression of proteins that have a role in plastids

Plastids play an integral role during plant development and it may therefore be of interest to express proteins (plant or non-plant) inside plastids that would have a positive effect on plant growth, development and/or confer modified characteristics to the plant as whole. Due to the high expression levels of transgenes in plastids coupled to the fact that the IPT as a selectable marker gene does not lead to the generation of spontaneous ribosomal mutants (as with the common spectinomycin selectable marker gene), the present invention represents an ideal system for this application.

The transgene is inserted into one of the pPTI001 or pPTI002 vectors (Figure 3 and 5) between the TrbcL and the TspbA polyA addition sequences in the pPTI001 vector series or between the Prrn promoter and the TspbA polyA addition sequence in the pPTI002 vector series and the construct transformed into plastids using the protocol shown in Appendix 1 followed by cytokinin-mediated selection and regeneration. Once regenerated, the IPT gene is removed by CRE-mediated recombination and the transgene is activated leading to minimal adverse effects on initial plant regeneration.

### Example 4

### High level expression of eukaryotic proteins

There are often problems associated with expression of eukaryotic proteins in bacterial systems due to insolubility and/or lack of post-translational modifications. Similarly, the expression of eukaryotic proteins in mammalian cells is expensive and labour intensive. The present system can be used for the expression of eukaryotic proteins in plastids using IPT marker gene selection and transgene activation.

As described in Examples 2 and 3, any gene encoding a eukaryotic protein may be inserted into one or all of the pPTI001 series or the pPTI002 series of vectors followed by transformation, selection and regeneration as described previously. Following regeneration, the expressed protein may be purified using the affinity tags shown in Figures 4 and 6 and used for downstream applications.

A non-exclusive list of possible eukaryotic protein families that will be expressed includes antibodies, enzymes, enzyme inhibitors and design peptides.

### Example 5

### High level expression of prokaryotic proteins

Due to the endosymbiotic origin of plastids, it is possible to express prokaryotic proteins in plastids. As described in Examples 2 and 3, any gene encoding a prokaryotic protein may be inserted into one or all of the pPTI001 series or the pPTI002 series of vectors followed by transformation, selection and regeneration as described previously. Following regeneration, the expressed protein may be purified using the affinity tags shown in Figures 4 and 6 and used for downstream applications.

### Example 6

### Coordinated expression of multiple proteins from the same promoter

Due to the endosymbiotic origin of plastids, it is possible to express operon-like gene structures in plastids. This entails the coordinated and simultaneous expression of multiple proteins under the regulation of a single promoter. As described in Examples 2 and 3, multiple genes encoding protein of both prokaryotic and eukaryotic origin (or a mix thereof) may be inserted into one or all of the pPTI001 series or the pPTI002 series of vectors followed by transformation, selection and regeneration as described previously.

### Example 7

### Expression of YFP from pPTI001/YFP in plastids of tobacco

pPTI001/YFP vector was bombarded into tobacco leaf cells and regenerants selected on media containing only auxin and on media lacking all hormones. Regenerants were obtained (Figure 8) and transferred to secondary selection media containing auxin to induce root formation before transfer to soil. The incorporation of the transformation cassette in the tobacco plastid genome was confirmed by PCR using vector-specific primers and primers in the flanking region of the tobacco chloroplast genome (Figure 9). Leaves from the regenerated plants were infiltrated with pER10/TP.CRE, a binary vector expressing the TP.CRE fusion, followed by induction. After 72 hours the infiltrated tissue was analysed by fluorescence microscopy revealing cells containing GFP fluorescing chloroplasts (Figure 10).

### Example 8

### Additional and new plastid transformation vectors

To optimize the selection and regeneration system further a new series of plastid transformation vectors have been constructed. The following modifications and additions have been made:
1) Inclusion of the aadA gene in addition to the IPT gene for double selection purposes.
2) Inclusion of new homologous recombination sites (HOM3 and HOM4) that may increase more efficient homologous recombination and transgene insertion.
3) Inclusion of an IPTG inducible promoter.
4) Inclusion of a modified Prrn promoter for higher expression levels.

The details of the vectors are shown in Figures 11-16. All the exemplified vectors contain the IPT selectable marker gene.

### Example 9

### Use of auxin biosynthesis as a selectable marker for plastid transformation

The plastid transformation vectors pPTA001-pPTA004 are constructed as detailed in Figures 18-21 using a 1289 bp homologous recombination sequence (104091-105380 nt) and a 989 bp homologous recombination sequence (105381-106370 nt) from the chloroplast genome from *Nicotiana tabacum* (Shinozaki K, Ohme M, Tanaka M, Wakasugi T, Hayashida N, Matsubayashi T, Zaita N, Chunwongse J, Obokata J, Yamaguchi-Shinozaki K, Ohto C, Torazawa K, Meng BY, Sugita M, Deno H, Kamogashira T, Yamada K, Kusuda J, Takaiwa F, Kato A, Tohdoh N, Shimada H, Sugiura M. (1986) The complete nucleotide sequence of the tobacco chloroplast genome: its gene organization and expression. EMBO J. 5, 2043-2049.) on either side of the gene cassette. Shorter or longer homologous recombination sequences are also used to ensure appropriate homologous recombination events.

These vectors are bombarded into tobacco leaves as detailed in Appendix 3. For transformation experiments, young actively growing leaves from plants growing in Magenta Box are used.

### Example 10

### Expression of proteins with toxic effects on plant development

The expression of high levels of foreign protein in plants can lead to detrimental effects on plant development because of toxic effects. The described system overcomes this by combining insertion of the transgene(s) into the plastid genome where it remains dormant until the auxin selectable marker genes are removed by CRE/lox mediated recombination.

The transgene encoding the "plant-toxic" protein is inserted into one of the pPTA001-pPTA004 vectors (Figures 18-21) between the Prrn promoter and the TpsbA polyA addition sequences and the construct transformed into plastids using the protocol shown in Appendix 3 followed by auxin-mediated selection and regeneration. Once regenerated, the auxin genes are removed by CRE-mediated recombination and the toxic transgene is activated leading to minimal adverse effects on initial plant regeneration. Once expressed, the transplastomic plants are used conferring a desired trait or the recombinant protein is purified.

### Example 11

### Expression of proteins that have a role in plastids

Plastids play an integral role during plant development and it may therefore be of interest to express proteins (plant or non-plant) inside plastids that would have a positive effect on plant growth, development and/or confer modified characteristics to the plant as whole. Due to the high expression levels of transgenes in plastids coupled to the fact that the iaaH and iaaM as selectable marker genes does not lead to the generation of spontaneous ribosomal mutants (as with the common spectinomycin selectable marker gene), the present invention represents an ideal system for this application.

The transgene encoding the plastidic protein is inserted into one of the pPTA001-pPTA004 vectors (Figures 18-21) between the Prrn promoter and the TrbcL polyA addition sequences and the construct transformed into plastids using the protocol shown in Appendix 3 followed by auxin-mediated selection and regeneration. Once regenerated, the iaaH and iaaM genes are removed by CRE-mediated recombination and the transgene is activated leading to minimal adverse effects on initial plant regeneration.

### Example 12

### High level expression of eukaryotic proteins

There are often problems associated with expression of eukaryotic proteins in bacterial systems due to insolubility and/or lack of post-translational modifications. Similarly, the expression of eukaryotic proteins in mammalian cells is expensive and labour intensive. The present system can be used for the expression of eukaryotic proteins in plastids using iaaH and iaaM marker gene selection and transgene activation.

As described in Examples 10 and 11, any gene encoding a eukaryotic protein may be inserted into one or all of pPTA001-pPTA004 series of vectors followed by transformation, selection and regeneration as described previously. Following regeneration, the expressed protein may be purified and used for downstream applications.

A non-exclusive list of possible eukaryotic protein families that will be expressed includes antibodies, enzymes, enzyme inhibitors and design peptides.

### Example 13

### High level expression of prokaryotic proteins

Due to the endosymbiotic origin of plastids, it is possible to express prokaryotic proteins in plastids. As described in Examples 10 and 11, any gene encoding a prokaryotic protein may be inserted into one or all of ppTA001-pTA004 vectors followed by transformation, selection and regeneration as described previously. Following regeneration, the expressed protein may be purified and used for downstream applications.

### Example 14

### Coordinated expression of multiple proteins from the same promoter

Due to the endosymbiotic origin of plastids, it is possible to express operon-like gene structures in plastids. This entails the coordinated and simultaneous expression of multiple proteins under the regulation of a single promoter. As described in Examples 10 and 11, multiple genes encoding protein of both prokaryotic and eukaryotic origin (or a mix thereof) may be inserted into one or all of the pPTA001-pPTA004 vectors followed by transformation, selection and regeneration as described previously.

### Example 15

### Additional and new plastid transformation vectors

To optimize the selection and regeneration system further a new series of plastid transformation vectors are constructed. The following modifications and additions are being made:
1) Inclusion of the aadA gene in addition to the iaaH and iaaM genes for double selection purposes (Figures 22-23, pPTA005 and pPTA006).
2) Inclusion of new homologous recombination sites with varying lengths and composition that may increase the efficiency of homologous recombination and transgene insertion.
3) Inclusion of a a variety of inducible promoters to drive transgene expression.
4) Inclusion of a modified Prrn promoter for higher expression levels. Other preferred constructs of the disclosure are given in Figures 24-26.

### Appendix 1

### PROTOCOL FOR CHLOROPLAST TRANSFORMATION USING CYTOKININ SELECTION

### TIME COURSE

The standard procedures produce transformed plants in 3-5 months.
- Bombardment to first shoot on: 3-8 weeks
- Subculture production of homoplastic plants: 3-4 weeks
- Growth in soil prior to analysis: 1-2 weeks

### EQUIPMENT SET UP

Helium Gun Biorad PDS 1000
- Rupture disk PSI: 1100
- Gap between rupture disk retaining cap and macrocarrier over cover lid: ¼"
- Spacer rings below stopping screen support: 2
- Level of macrocarrier launch assembly: 1 (from top)
- Level of Petri dish holder: 4 (from top)
- Vacuum inflow rate: Maximum
- Vacuum release rate: Attenuate the release so it approximates the speed of vacuum inflow.

### STOCK SOLUTIONS

- 2.5 M CaCl₂ autoclave or filter sterilize
- 1 M Spermidine Free Base in sterilize H₂O
- dH₂O
- DNA at 1 µg/µl in dH₂O or 1X TE
- 100% Ethanol
- 70% Ethanol

### CONSUMABLES

- 1100 psi rupture disks
- Stopping screens
- Macrocarriers
- Gold particles

### PREPARATION OF THE DNA-GOLD PARTICLE MIX

- 50 mg of Gold particles are suspended in 1 ml of absolute ethanol as stock.
- Take 0.25 ml of Gold stock suspension and microfuge for 5 seconds. Remove Ethanol and wash 3 times with sterile distilled H₂O, microfuging 3 minutes between washings.
- Resuspend Gold in 0.25 ml dH₂O.
- Aliquot 50 µl of Gold-H₂O suspension into Eppendorf tubes.
- Into each Eppendorf add the following in succession:
   10 µl DNA at 1 µlg/µl
   50 µl of 2.5 M CaCl₂
   20 µl of 0.1 M Spermidine free base
- Vortex for 5 minutes at highest speed.
- Add 200 µl of absolute ethanol to each tube.
- Spin in microfuge at 3000 rpm for 2 seconds.
- Remove supernatant as much as possible and rinse pellet in absolute ethanol once, microfuging at 3000 rpm for 2 seconds.
- Resuspend pellet in 30 µl absolute ethanol (makes 4-5 shots). Store mixture on ice.

### PREPARING THE BIOLISTIC GUN AND CONSUMABLES

- Sterilize the gun vacuum chamber and surfaces with 70% ethanol or 70% isopropanol.
- Sterilize the rupture disks and macrocarrier holders in 70% ethanol for 10 minutes. Air dry in hood.
- Soak the macrocarriers in absolute ethanol to remove all traces of H₂O. Air dry in hood.
- Open the helium tank. Set the helium tank regulator to 1300 psi (or 200 psi over the rating of the rupture disks).

### BOMBARDMENT

- Snap the macrocarriers into their holders.
- Pipet 5 µl of the gold-DNA mixture onto the centre of the macrocarrier. The mixture should spread out evenly with few chunks.
- Place a rupture disk in the holder ring and tighten ring to the helium barrel.
- Place a stopping screen and macrocarrier with gold-DNA (in holder) into the retaining assembly and screw down.
- Place assembly into the vacuum chamber at level 1 (first from top).
- Place sample at level 4. Remove Petri dish lid.
- Turn on vacuum pump.
- Pull a vacuum to 27-29 in.Hg.
- Press and hold fire button till the rupture disk breaks.
- Release vacuum and remove sample.
- Remove rupture disk, macrocarrier and stopping screen.
- Repeat if desired.
- At the end of the experiment, turn off the helium tank. Pull a vacuum in the gun and release the remaining helium through the gun, then turn off the helium regulator.

The key to successful bombardment is usually in the spread of particles on the macrocarrier. The ethanol/gold/DNA mixture should quickly spread out over the centre of the macrocarrier. The resulting spread should be a very fine dusting of particles, evenly spread and containing few chunks. Chunk causes increased cell death.

Each 30 µl Gold-DNA mix gives four or five bombardments. The remaining mixture is usually too dense for good results.

**TOBACCO PREPARATION AND REGENERATION MEDIA**

| | | |
|---|---|---|
| MS: | MS salts and vitamins (1X) | |
| | 30 g/l sucrose | |
| | 6 g/l phytagar | |
| | pH 5.8 | Autoclave |
| | | |
| RMOP: | MS salts (1X) | |
| | 1 mg/l BAP | |
| | 0.1 mg/l NAA | |
| | 1 mg/l Thiamine | |
| | 100 mg/l inositol | |
| | 30 g/l sucrose | |
| | 6 g/l phytagar | |
| | pH 5.8 | Autoclave |
| | | |
| RMOP- BAP | | |
| | pH 5.8 | Autoclave |
| | | |
| MS + | MS media | |
| | 1 mg/l IBA (indole-3-butyric acid) | |
| | 2 µM 17-β-estradiol | |
| | pH 5.8 | Autoclave |

### TISSUE CULTURE

- Tobacco plants are micropropagated using sterile technique in magenta boxes containing MS media.
- Expanded leaves are excised and placed abaxial surface up on a Whatman filter paper laying on top of RMOP media. The leaves are allowed to desiccate slightly (1-2 hours) prior to bombardment on the filter paper. Each bombardment can treat 1-3 leaves covering approx. 1/3 of the 9 cm Petri dish.
- Post bombardment, the plates are sealed and the leaves are left at 12:12 photoperiod at 24°C for 2 days.
- After two days the leaves are cut into sections approx. 5 mm square and placed abaxial side down on RMOP media lacking BAP.
- Green shoots can be collected from the bleached explants in 3-8 weeks.
- Leaves from these shoots are cut up (2mm square) and subcultured in the same selective media for approx. 4 weeks.
- Typically 4 shoots are collected per initial subcultured shoots. These are rooted in tubes containing MS media + 1mg/l IBA and 2 µM 17-β-estradiol.
- Rooted shoots are transferred to soil approx. 3-5 weeks after isolation. Plants are allowed to grow in standard tobacco conditions (16:8 photoperiod at 25°C).

### NOTES

1. Tobacco leaves do not have to lay completely flat for bombardment.
2. Tobacco leaves will lose their turgor after 2 days on filter paper. This is OK.
3. Do minimum amount of transferring. The maximum amount of transferring that needs to be done:
   a. Excise leaves from plants grown in MS. Place leaves on filter paper on RMOP.
   b. 2 days post bombardment place leaf explants on RMOP - BAP
   c. 3-8 weeks later, remove leaves from green shoots, cut, then transfer to RMOP - BAP.
   d. Collect 4 shoots, transfer to MS+ 1mg/l IBA+ 2 µM 17-β-estradiol in individual tubes.
4. Transformation frequency for an average experiment is anywhere from 1.5 stably transformed plants to 0.3 stably transformed plants per bombardment.

**MEDIA FOR TOBACCO CHLOROPLAST TRANSFORMATION**

| **Name** | **Compositions** | **Container** | **Time⁴** |
|---|---|---|---|
| MSS: | MS Salts and Vitamins (1X) | Magenta Box | |
| | 30 g/l Sucrose | | |
| | 6 g/l Phytagar | | |
| | pH 5.8 | | |
| | Autoclave | | |
| | | | |
| MFB¹ | MS Salts (1X) | Petri Dish (9 cm) | 2 days |
| | 1 mg/l BAP | | |
| | 0.1 mg/l NAA | | |
| | 1 mg/l Thiamine | | |
| | 100 mg/l Inositol | | |
| | 30 g/l Sucrose | | |
| | 6 g/l Phytagar | | |
| | pH 5.8 | | |
| | Autoclave | | |
| | | | |
| MTS² | MFB minus BAP | Deep Petri Dish | 3-10 weeks⁵ |
| | | | |
| MFR³ | MSS | Magenta Box | 3-5 weeks |
| | 1 mg/l IBA (indole-3-butyric acid) | | |
| | 2 µM 17-β-estradiol | | |

| | | | |
|---|---|---|---|
| Notes: 1. MFB: media for bombardement 2. MTS: media for transgenic selection 3. MFR: media for rooting 4. Time: time that tobacco leaves stay in the media 5. This time includes second selection time | | | |

### Appendix 2

### PROTOCOL FOR PLASTID TRANSFORMATION USING CYTOKININ AND ANTIBIOTICS SELECTION IN COMBINATION EQUIPMENT SET UP

Helium Gun Biorad PDS 1000
- Rupture disk PSI: 1100
- Gap between rupture disk retaining cap and macrocarrier over cover lid: ¼"
- Spacer rings below stopping screen support: 2
- Level of macrocarrier lauch assembly: 1 (from top)
- Level of Petri dish holder: 4 (from top)
- Vacuum inflow rate: Maximum
- Vacuum release rate: Attenuate the release so it approximates the speed of vaccum inflow.

### STOCK SOLUTIONS

- 2.5 M CaCl₂ autocalve or filter sterilize
- 1 M Spermidine Free Base in sterilize H₂O
- dH₂O
- DNA at 1 µg/ul in dH₂O or 1X TE
- 100% Ethanol
- 70% Ethanol

### CONSUMABLES

- 1100 psi rupture disks
- Stopping screens
- Macrocarriers
- Gold particles

### PREPARATION OF THE DNA-GOLD PARTICLE MIX

- 50 mg of Gold particles are suspended in 1 ml of absolute ethanol as stock.
- Take 0.25 ml of Gold stock suspension and microfuge for 5 seconds. Remove Ethanol and wash 3 times with sterile distilled H₂O, microfuging 3 minutes between washings.
- Resuspend Gold in 0.25 ml dH₂O.
- Aligquot 50 µl of Gold-H₂O suspendsion into Eppendorf tubes.
- Into each Eppendorf add the following in succession:
   10 µl DNA at 1 µg/µl
   50 µl of 2.5 M CaCl₂
   20 µl of 0.1 M Spermidine free base
- Vortex for 5 minutes at highest speed.
- Add 200 µl of absolute ethanol to each tube.
- Spin in microfuge at 3000 rpm for 2 seconds.
- Remove supernatant as much as possible and rinse pellet in absolute ethanol once, microfuging at 3000 rpm for 2 seconds.
- Resuspend pellet in 30 µl absolute ethanol (makes 4-5 shots). Store mixture on ice.

### PREPARING THE BIOLISTIC GUN AND CONSUMABLES

- Sterilize the gun vaccum chamber and surfaces with 70% ethanol or 70% isopropanol.
- Sterilize the rupture disks and macrocarrier holders in 70% ethanol for 10 minutes. Air dry in hood.
- Soak the macrocarriers in absolute ethanol to remove all traces of H₂O. Air dry in hood.
- Open the helium tank. Set the helium tank regulator to 1300 psi (or 200 psi over the rating of the rupture disks).

### BOMBARDMENT

- Snap the macrocarriers into their holders.
- Pipet 5 µl of the gold-DNA mixture onto the centre of the macrocarrier. The mixture should spread out evenly with few chunks.
- Place a rupture disk in the holder ring and tighten ring to the helium barrel.
- Place a stopping screen and macrocarrier with gold-DNA (in holder) into the retaining assembly and screw down.
- Place assembly into the vacuum chamber at level 1 (first from top).
- Place sample at level 4. Remove Petri dish lid.
- Turn on vacuum pump.
- Pull a vacuum to 27-29 in.Hg.
- Press and hold fire button till the rupture disk breaks.
- Release vacuum and remove sample.
- Remove rupture disk, macrocarrier and stopping screen.
- Repeat if desired.
- At the end of the experiment, TURN OFF THE HELIUM TANK. Pull a vacuum in the gun and release the remaining helium through the gun, then turn off the helium regulator.

### NOTE

1. The key to successful bombardment is usually in the spread of particles on the macrocarrier. The ethanol/gold/DNA mixture should quickly spread out over the centre of the macrocarrier. The resulting spread should be a very fine dusting of particles, evenly spread and containing few chunks. And chunk causes cell death.
2. Each 30 µl Gold-DNA mix gives four or five bombardments. The remaining mixture is usually too dense for good results.

**TOBACCO PREPARATION AND REGENERATION MEDIA**

| | | |
|---|---|---|
| MS: | MS salts and vitamins (1X) | |
| | 30 g/l sucrose | |
| | 6 g/l phytagar | |
| | pH 5.8 | Autoclave |
| | | |
| RMOP: | MS salts (1X) | |
| | 1 mg/l BAP | |
| | 0.1 mg/l NAA | |
| | 1 mg/l Thiamine | |
| | 100 mg/l inositol | |
| | 30 g/l sucrose | |
| | 6 g/l phytagar | |
| | pH 5.8 | Autoclave |
| | | |
| RMOP- BAP + Spectinomycin | | |
| | 500 µg/ml Spectinomycin | |
| | pH 5.8 | Autoclave |
| | | |
| MS + | MS media | |
| | 1 mg/l IBA (indole-3-butyric acid) | |
| | 2 µM 17-β-estradiol | |
| | pH 5.8 | Autoclave |

### TISSUE CULTURE

- Tobacco plants are micropropagated using sterile technique in magenta boxes containing MS media
- Expanded leaves are excised and placed abaxial surface up on a Whatman filter paper laying on top of RMOP media. The leaves are allowed to desiccate slightly (1-2 hours) prior to bombardment on the filter paper. Each bombardment can treat 1-3 leaves covering approx. 1/3 of the 9 cm Petri dish.
- Post bombardment, the plates are sealed and the leaves are left at 12:12 photoperiod at 24°C for 2 days.
- After two days the leaves are cut into sections approx. 5 mm square and placed abaxial side down on RMOP media lacking BAP and containing spectinomycin.
- Green shoots can be collected from the bleached explants in 3-8 weeks.
- Leaves from these shoots are cut up (2mm square) and subcultured in the same selective media for approx. 4 weeks.
- Typically 4 shoots are collected per initial subcultured shoots. These are rooted in tubes containing MS media + 1mg/l IBA and 2 µM 17-β-estradiol.
- Rooted shoots are transferred to soil appox. 3-5 weeks after isolation. Plants are allowed to grow in standard tobacco conditions (16:8 photoperiod at 25°C).

### NOTE

1. Tobacco leaves do not have to lay completely flat for bombardment.
2. Tobacco leaves will lose their turgor after 2 days on filter paper. This is OK.
3. Do minimum amount of transferring. The maximum amount of transferring that needs to be done:
   a. Excise leaves from plants grown in MS. Place leaves on filter paper on RMOP.
   b. 2 days post bombardment place leaf explants on RMOP - BAP + Spectinomycin
   c. 3-8 weeks later, remove leaves from green shoots, cut, then transfer to RMOP - BAP + Spectinomycin.
   d. Collect 4 shoots, transfer to MS+ 1mg/l IBA+ 2 µM 17-β-estradiol in individual tubes.
4. Transformation frequency for an average experiment is anywhere from 1.5 stably transformed plants to 0.3 stably transformed plants per bombardment.

**MEDIA FOR TOBACCO CHLOROPLAST TRANSFORMATION**

| **Name** | **Compostions** | **Container** | **Time⁴** |
|---|---|---|---|
| MSS: | MS Salts and Vitamins (1X) | Magenta Box | |
| | 30 g/l Sucrose | | |
| | 6 g/l Phytagar | | |
| | pH 5.8 | | |
| | Autoclave | | |
| | | | |
| MFB¹ | MS Salts (1X) | Petri Dish (9 cm) | 2 days |
| | 1 mg/l BAP | | |
| | 0.1 mg/l NAA | | |
| | 1 mg/l Thiamine | | |
| | 100 mg/l Inositol | | |
| | 30 g/l Sucrose | | |
| | 6 g/l Phytagar | | |
| | pH 5.8 | | |
| | Autoclave | | |
| | | | |
| MTS² | MFB minus BAP + Spectinomycin | Deep Petri Dish | 3-10 weeks⁵ |
| | | | |
| MFR³ | MSS | Magenta Box | 3-5 weeks |
| | 1 mg/l IBA (indole-3-butyric acid) | | |
| | 2 µM 17-β-estradiol | | |

| | | | |
|---|---|---|---|
| Note: 1. MFB: media for bombardment 2. MTS: media for transgenic selection 3. MFR: media for rooting 4. Time: time that tobacco leaves stay in the media 5. This time includes second selection time | | | |

### Appendix 3

### PROTOCOL FOR PLASTID TRANSFORMATION USING AUXIN SELECTION

### EQUIPMENT SET UP

Helium Gun Biorad PDS 1000
- Rupture disk PSI: 1100
- Gap between rupture disk retaining cap and macrocarrier over cover lid: ¼"
- Spacer rings below stopping screen support: 2
- Level of macrocarrier lauch assembly: 1 (from top)
- Level of Petri dish holder: 4 (from top)
- Vacuum inflow rate: Maximum
- Vacuum release rate: Attenuate the release so it approximates the speed of vaccum inflow.

### STOCK SOLUTIONS

- 2.5 M CaCl₂ autocalve or filter sterilize
- 1 M Spermidine Free Base in sterilize H₂O
- dH₂O
- DNA at 1 µg/ul in dH₂O or 1X TE
- 100% Ethanol
- 70% Ethanol

### CONSUMABLES

- 1100 psi rupture disks
- Stopping screens
- Macrocarriers
- Gold particles

### PREPARATION OF THE DNA-GOLD PARTICLE MIX

- 50 mg of Gold particles are suspended in 1 ml of absolute ethanol as stock.
- Take 0.25 ml of Gold stock suspension and microfuge for 5 seconds. Remove Ethanol and wash 3 times with sterile distilled H₂O, microfuging 3 minutes between washings.
- Resuspend Gold in 0.25 ml dH₂O.
- Aligquot 50 µl of Gold-H₂O suspendsion into Eppendorf tubes.
- Into each Eppendorf add the following in succession:
   10 µl DNA at 1 µg/µl
   50 µl of 2.5 M CaCl₂
   20 µl of 0.1 M Spermidine free base
- Vortex for 5 minutes at highest speed.
- Add 200 µl of absolute ethanol to each tube.
- Spin in microfuge at 3000 rpm for 2 seconds.
- Remove supernatant as much as possible and rinse pellet in absolute ethanol once, microfuging at 3000 rpm for 2 seconds.
- Resuspend pellet in 30 µl absolute ethanol (makes 4-5 shots). Store mixture on ice.

### PREPARING THE BIOLISTIC GUN AND CONSUMABLES

- Sterilize the gun vaccum chamber and surfaces with 70% ethanol or 70% isopropanol.
- Sterilize the rupture disks and macrocarrier holders in 70% ethanol for 10 minutes. Air dry in hood.
- Soak the macrocarriers in absolute ethanol to remove all traces of H₂O. Air dry in hood.
- Open the helium tank. Set the helium tank regulator to 1300 psi (or 200 psi over the rating of the rupture disks).

### BOMBARDMENT

- Snap the macrocarriers into their holders.
- Pipet 5 µl of the gold-DNA mixture onto the centre of the macrocarrier. The mixture should spread out evenly with few chunks.
- Place a rupture disk in the holder ring and tighten ring to the helium barrel.
- Place a stopping screen and macrocarrier with gold-DNA (in holder) into the retaining assembly and screw down.
- Place assembly into the vacuum chamber at level 1 (first from top).
- Place sample at level 4. Remove Petri dish lid.
- Turn on vacuum pump.
- Pull a vacuum to 27-29 in.Hg.
- Press and hold fire button till the rupture disk breaks.
- Release vacuum and remove sample.
- Remove rupture disk, macrocarrier and stopping screen.
- Repeat if desired.
- At the end of the experiment, TURN OFF THE HELIUM TANK. Pull a vacuum in the gun and release the remaining helium through the gun, then turn off the helium regulator.

### NOTE

1. The key to successful bombardment is usually in the spread of particles on the macrocarrier. The ethanol/gold/DNA mixture should quickly spread out over the centre of the macrocarrier. The resulting spread should be a very fine dusting of particles, evenly spread and containing few chunks. And chunk causes cell death.
2. Each 30 µl Gold-DNA mix gives four or five bombardments. The remaining mixture is usually too dense for good results.

**TOBACCO PREPARATION AND REGENERATION MEDIA**

| | | |
|---|---|---|
| MS: | MS salts and vitamins (1X) | |
| | 30 g/l sucrose | |
| | 6 g/l phytagar | |
| | pH 5.8 | Autoclave |
| | | |
| RMOP: | MS salts (1X) | |
| | 1 mg/l BAP | |
| | 0.1 mg/l NAA | |
| | 1 mg/l Thiamine | |
| | 100 mg/l inositol | |
| | 30 g/l sucrose | |
| | 6 g/l phytagar | |
| | pH 5.8 | Autoclave |
| RMOP- NAA | | |
| | pH 5.8 | Autoclave |
| | | |
| MS + | MS media | |
| | 1 mg/l IBA (indole-3-butyric acid) | |
| | 2 µM 17-β-estradiol | |
| | pH 5.8 | Autoclave |

### TISSUE CULTURE

- Tobacco plants are micropropagated using sterile technique in magenta boxes containing MS media
- Expanded leaves are excised and placed abaxial surface up on a Whatman filter paper laying on top of RMOP media. The leaves are allowed to desiccate slightly (1-2 hours) prior to bombardment on the filter paper. Each bombardment can treat 1-3 leaves covering approx. 1/3 of the 9 cm Petri dish.
- Post bombardment, the plates are sealed and the leaves are left at 12:12 photoperiod at 24°C for 2 days.
- After two days the leaves are cut into sections approx. 5 mm square and placed abaxial side down on RMOP media lacking NAA.
- Green shoots can be collected from the bleached explants in 3-8 weeks.
- Leaves from these shoots are cut up (2mm square) and subcultured in the same selective media for approx. 4 weeks.
- Typically 4 shoots are collected per initial subcultured shoots. These are rooted in tubes containing MS media + 1mg/l IBA and 2 µM 17-β-estradiol.
- Rooted shoots are transferred to soil approx. 3-5 weeks after isolation. Plants are allowed to grow in standard tobacco conditions (16:8 photoperiod at 25°C).

### NOTE

1. Tobacco leaves do not have to lay completely flat for bombardment.
2. Tobacco leaves will lose their turgor after 2 days on filter paper. This is OK.
3. Do minimum amount of transferring. The maximum amount of transferring that needs to be done:
   a. Excise leaves from plants grown in MS. Place leaves on filter paper on RMOP.
   b. 2 days post bombardment place leaf explants on RMOP - NAA
   c. 3-8 weeks later, remove leaves from green shoots, cut, then transfer to RMOP - NAA.
   d. Collect 4 shoots, transfer to MS+ 1mg/l IBA+ 2 µM 17-β-estradiol in individual tubes.
4. Transformation frequency for an average experiment is anywhere from 1.5 stably transformed plants to 0.3 stably transformed plants per bombardment.

**MEDIA FOR TOBACCO CHLOROPLAST TRANSFORMATION**

| **Name** | **Compostions** | **Container** | **Time⁴** |
|---|---|---|---|
| MSS: | MS Salts and Vitamins (1X) | Magenta Box | |
| | 30 g/l Sucrose | | |
| | 6 g/l Phytagar | | |
| | pH 5.8 | | |
| Autoclave | | | |
| | | | |
| MFB¹ | MS Salts (1X) | Petri Dish (9 cm) | 2 days |
| | 1 mg/l BAP | | |
| | 0.1 mg/l NAA | | |
| | 1 mg/l Thiamine | | |
| | 100 mg/l Inositol | | |
| | 30 g/l Sucrose | | |
| | 6 g/l Phytagar | | |
| | pH 5.8 | | |
| | Autoclave | | |
| | | | |
| MTS² | MFB minus NAA | Deep Petri Dish | 3-10 weeks⁵ |
| | | | |
| MFR³ | MSS | Magenta Box | 3-5 weeks |
| | 1 mg/l IBA (indole-3-butyric acid) | | |
| | 2 µM 17-β-estradiol | | |

| | | | |
|---|---|---|---|
| Note: 1. MFB: media for bombardment 2. MTS: media for transgenic selection 3. MFR: media for rooting 4. Time: time that tobacco leaves stay in the media 5. This time includes second selection time | | | |

### Appendix 4

### PROTOCOL FOR PLASTID TRANSFORMATION USING AUXIN AND ANTIBIOTIC SELECTION IN COMBINATION

### EQUIPMENT SET UP

Helium Gun Biorad PDS 1000
- Rupture disk PSI: 1100
- Gap between rupture disk retaining cap and macrocarrier over cover lid: ¼"
- Spacer rings below stopping screen support: 2
- Level of macrocarrier lauch assembly: 1 (from top)
- Level of Petri dish holder: 4 (from top)
- Vacuum inflow rate: Maximum
- Vacuum release rate: Attenuate the release so it approximates the speed of vaccum inflow.

### STOCK SOLUTIONS

- 2.5 M CaCl₂ autocalve or filter sterilize
- 1 M Spermidine Free Base in sterilize H₂O
- dH₂O
- DNA at 1 µg/ul in dH₂O or 1X TE
- 100% Ethanol
- 70% Ethanol

### CONSUMABLES

- 1100 psi rupture disks
- Stopping screens
- Macrocarriers
- Gold particles

### PREPARATION OF THE DNA-GOLD PARTICLE MIX

- 50 mg of Gold particles are suspended in 1 ml of absolute ethanol as stock.
- Take 0.25 ml of Gold stock suspension and microfuge for 5 seconds. Remove Ethanol and wash 3 times with sterile distilled H₂O, microfuging 3 minutes between washings.
- Resuspend Gold in 0.25 ml dH₂O.
- Aligquot 50 µul of Gold-H₂O suspendsion into Eppendorf tubes.
- Into each Eppendorf add the following in succession:
   10 µl DNA at 1 µg/µl
   50 µl of 2.5 M CaCl₂
   20 µl of 0.1 M Spermidine free base
- Vortex for 5 minutes at highest speed.
- Add 200 µl of absolute ethanol to each tube.
- Spin in microfuge at 3000 rpm for 2 seconds.
- Remove supernatant as much as possible and rinse pellet in absolute ethanol once, microfuging at 3000 rpm for 2 seconds.
- Resuspend pellet in 30 µl absolute ethanol (makes 4-5 shots). Store mixture on ice.

### PREPARING THE BIOLISTIC GUN AND CONSUMABLES

- Sterilize the gun vacuum chamber and surfaces with 70% ethanol or 7 0% isopropanol.
- Sterilize the rupture disks and macrocarrier holders in 70% ethanol for 10 minutes. Air dry in hood.
- Soak the macrocarriers in absolute ethanol to remove all traces of H₂O. Air dry in hood.
- Open the helium tank. Set the helium tank regulator to 1300 psi (or 200 psi over the rating of the rupture disks).

### BOMBARDMENT

- Snap the macrocarriers into their holders.
- Pipet 5 µl of the gold-DNA mixture onto the centre of the macrocarrier. The mixture should spread out evenly with few chunks.
- Place a rupture disk in the holder ring and tighten ring to the helium barrel.
- Place a stopping screen and macrocarrier with gold-DNA (in holder) into the retaining assembly and screw down.
- Place assembly into the vacuum chamber at level 1 (first from top).
- Place sample at level 4. Remove Petri dish lid.
- Turn on vacuum pump.
- Pull a vacuum to 27-29 in.Hg.
- Press and hold fire button till the rupture disk breaks.
- Release vacuum and remove sample.
- Remove rupture disk, macrocarrier and stopping screen.
- Repeat if desired.
- At the end of the experiment, TURN OFF THE HELIUM TANK. Pull a vacuum in the gun and release the remaining helium through the gun, then turn off the helium regulator.

### NOTE

1. The key to successful bombardment is usually in the spread of particles on the macrocarrier. The ethanol/gold/DNA mixture should quickly spread out over the centre of the macrocarrier. The resulting spread should be a very fine dusting of particles, evenly spread and containing few chunks. And chunk causes cell death.
2. Each 30 µl Gold-DNA mix gives four or five bombardments. The remaining mixture is usually too dense for good results.

**TOBACCO PREPARATION AND REGENERATION MEDIA**

| | | |
|---|---|---|
| MS: | MS salts and vitamins (1X) | |
| | 30 g/l sucrose | |
| | 6 g/l phytagar | |
| | pH 5.8 | Autoclave |
| | | |
| RMOP: | MS salts (1X) | |
| | 1 mg/l BAP | |
| | 0.1 mg/l NAA | |
| | 1 mg/l Thiamine | |
| | 100 mg/l inositol | |
| | 30 g/l sucrose | |
| | 6 g/l phytagar | |
| | pH 5.8 | Autoclave |
| RMOP- NAA + Spectinomycin | | |
| | 500 µg/ml Spectinomycin | |
| | pH 5.8 | Autoclave |
| | | |
| MS + | MS media | |
| | 1 mg/l IBA (indole-3-butyric acid) | |
| | 2 µM 17-β-estradiol | |
| | pH 5.8 | Autoclave |

### TISSUE CULTURE

- Tobacco plants are micropropagated using sterile technique in magenta boxes containing MS media
- Expanded leaves are excised and placed abaxial surface up on a Whatman filter paper laying on top of RMOP media. The leaves are allowed to desiccate slightly (1-2 hours) prior to bombardment on the filter paper. Each bombardment can treat 1-3 leaves covering approx. 1/3 of the 9 cm Petri dish.
- Post bombardment, the plates are sealed and the leaves are left at 12:12 photoperiod at 24°C for 2 days.
- After two days the leaves are cut into sections approx. 5 mm square and placed abaxial side down on RMOP media lacking NAA and containing spectinomycin.
- Green shoots can be collected from the bleached explants in 3-8 weeks.
- Leaves from these shoots are cut up (2mm square) and subcultured in the same selective media for approx. 4 weeks.
- Typically 4 shoots are collected per initial subcultured shoots. These are rooted in tubes containing MS media + 1mg/l IBA and 2 µM 17-β-estradiol.
- Rooted shoots are transferred to soil appox. 3-5 weeks after isolation. Plants are allowed to grow in standard tobacco conditions (16:8 photoperiod at 25°C).

### NOTE

1. Tobacco leaves do not have to lay completely flat for bombardment.
2. Tobacco leaves will lose their turgor after 2 days on filter paper. This is OK.
3. Do minimum amount of transferring. The maximum amount of transferring that needs to be done:
   a. Excise leaves from plants grown in MS. Place leaves on filter paper on RMOP.
   b. 2 days post bombardment place leaf explants on RMOP - NAA + Spectinomycin
   c. 3-8 weeks later, remove leaves from green shoots, cut, then transfer to RMOP - NAA + Spectinomycin.
   d. Collect 4 shoots, transfer to MS+ 1mg/l IBA+ 2 µM 17-β-estradiol in individual tubes.
4. Transformation frequency for an average experiment is anywhere from 1.5 stably transformed plants to 0.3 stably transformed plants per bombardment.

**MEDIA FOR TOBACCO CHLOROPLAST TRANSFORMATION**

| **Name** | **Compositions** | **Container** | **Time⁴** |
|---|---|---|---|
| MSS: | MS Salts and Vitamins (1X) | Magenta Box | |
| | 30 g/l Sucrose | | |
| | 6 g/l Phytagar | | |
| | pH 5.8 | | |
| Autoclave | | | |
| | | | |
| MFB1 | MS Salts (1X) | Petri Dish (9 cm) | 2 days |
| | 1 mg/l BAP | | |
| | 0.1 mg/l NAA | | |
| | 1 mg/l Thiamine | | |
| | 100 mg/l Inositol | | |
| | 30 g/l Sucrose | | |
| | 6 g/l Phytagar | | |
| | pH 5.8 | | |
| | Autoclave | | |
| | | | |
| MTS2 | MFB minus NAA + Spectinomycin | Deep Petri Dish | 3-10 weeks⁵ |
| MFR3 | MSS | Magenta Box | 3-5 weeks |
| 1 mg/l IBA (indole-3-butyric acid) | | | |
| 2 µM 17-β-estradiol | | | |

| | | | |
|---|---|---|---|
| Note: 1. MFB: media for bombardment 2. MTS: media for transgenic selection 3. MFR: media for rooting 4. Time: time that tobacco leaves stay in the media 5. This time includes second selection time | | | |

## Claims

1. A method for producing a transformed plant cell, the method comprising the step:
(i) transforming the plant cell with a genetic construct,
wherein the genetic construct comprises first and second homologous recombination elements flanking a Transformation Cassette,
wherein the first and second homologous recombination elements are capable of directing the integration of the Transformation Cassette into the genome of at least one plastid which is present in the plant cell,
wherein the Transformation Cassette comprises:
(a) a first promoter which is operable in said plant cell,
(b) an Excision Cassette,
(c) one or more transgenes,
(d) a first terminator element,
wherein the Excision Cassette comprises:
(b1) a first site-specific recombination element,
(b2) an optional second promoter
(b3) one or more nucleotide sequences encoding one or more plant-auxin biosynthetic polypeptides, wherein the plant-auxin biosynthetic polypeptides are selected from iaaH (indoleacetamide hydrolase) and iaaM (tryptophan mono-oxygenase),
(b4) a second terminator element,
(b5) a second site-specific recombination element,
wherein the first and second site-specific recombination elements are lox sites and are capable of being recognised by a recombinase, wherein the recombinase is a Cre recombinase,
preferably wherein the method additionally comprises the step:
(ii) selecting for transformed plant cells on media which is lacking auxin, and/or
(iii) expressing a recombinase in the plant cell, wherein the recombinase is one which recognises the first and second site-specific recombination elements.

2. A method as claimed in claim 1:
(A) wherein at least one of the one or more transgenes codes for an antibody, antibiotic, herbicide, vaccine antigen, enzyme, enzyme inhibitor or design peptide; and/or
(B) wherein the nucleotide sequences of the homologous recombination elements are selected such that the Transformation Cassette is specifically targeted to one or more selected plastids; and/or
(C) wherein the nucleotide sequences of the homologous recombination elements are selected such that no or essentially no Transformation Cassettes become integrated into the nuclear genome of the plant; and/or
(D) wherein the Transformation Cassette additionally comprises a nucleotide sequence encoding a polypeptide which confers resistance to an antibiotic, preferably wherein the antibiotic is spectinomycin.

3. A method as claimed in any one of the preceding claims, wherein the plant is a monocot or dicot, preferably wherein the plant is selected from the group consisting of cereals (rice, wheat, barley, oats, sorghum, corn), legumes (alfalfa, lentils, peanut, pea, soybean), oil crops (palm, sunflower, coconut, canola, olive), cash crops (cotton, sugar cane, cassava), vegetable crops (potato, tomato, carrot, sweet potato, sugar-beet, squash, cucumber, lettuce, broccoli, cauliflower, snap bean, cabbage, celery, onion, garlic), fruits/trees and nuts (banana, grape cantaloupe, muskmelon, watermelon, strawberry, orange, apple, mango, avocado, peach, grapefruit, pineapple, maple, almond), beverages (coffee, tea, cocoa), timber trees (oak, black walnut, sycamore), moss and duckweed, more preferably wherein the plant is tobacco or lettuce.

4. A method as claimed in any one of the preceding claims, wherein the plant cells are individual cells, groups of cells, in dissociated form or undissociated form, as part of a plant tissue or plant part, or a protoplast or a plant liquid culture; and/or wherein the plant cells are present in plant leaves.

5. A method as claimed in any one of the preceding claims, wherein the plastid is selected from chloroplasts, leucoplasts, amyloplasts, etioplasts, chromoplasts, elaioplasts and gerontoplasts, preferably, wherein the plastid is a green plastid, more preferably wherein the plastid is a chloroplast.

6. A method for producing a plant, comprising producing a plant cell by a method as defined in any one of the preceding claims, and regenerating a plant from the plant cell.

7. A method of producing a transgene product, comprising a method as defined in any one of the preceding claims, and additionally comprising the step of purifying or isolating, and optionally packaging, the transgene product, preferably wherein the transgene product is an antibody, antibiotic, herbicide, vaccine antigen, enzyme, enzyme inhibitor or design peptide.

8. A genetic construct comprising first and second homologous recombination elements flanking a Transformation Cassette,
wherein the first and second homologous recombination elements are capable of directing the integration of the Transformation Cassette into the genome of at least one plastid which is present in a plant cell,
wherein the Transformation Cassette comprises:
(a) a first promoter which is operable in a plant cell,
(b) an Excision Cassette,
(c) one or more transgenes,
(d) a first terminator element,
wherein the Excision Cassette comprises:
(b1) a first site-specific recombination element,
(b2) an optional second promoter
(b3) one or more nucleotide sequences encoding one or more plant-auxin biosynthetic polypeptides, wherein the plant-auxin biosynthetic polypeptides are selected from iaaH (indoacetamide hydrolase) and iaaM (tryptophan mono-oxygenase),
(b4) a second terminator element,
(b5) a second site-specific recombination element,
wherein the first and second site-specific recombination elements are capable of being recognised by a recombinase,
wherein the first and second site-specific recombination elements are lox sites, and wherein the recombinase is a Cre recombinase.

9. A genetic construct as claimed in claim 8:
(A) wherein the nucleotide sequences of the homologous recombination elements are selected such that the Transformation Cassette is specifically targeted to one or more selected plastids; or
(B) wherein the nucleotide sequences of the homologous recombination elements are selected such that no or essentially no Transformation Cassettes become integrated into the nuclear genome of the plant.

10. A plant cell or a plant or a plant seed comprising a genetic construct as claimed in any one of claims 8 or 9.

11. A method of producing a transgene product, comprising purifying or isolating, and optionally packaging, a transgene product from the plant cell, plant or plant seed of claim 10,
preferably wherein the transgene product is an antibody, antibiotic, herbicide, vaccine antigen, enzyme, enzyme inhibitor or design peptide.

## Patentansprüche

1. Verfahren zur Herstellung einer transformierten Pflanzenzelle, wobei das Verfahren den folgenden Schritt umfasst:
(i) Transformieren der Pflanzenzelle mit einem genetischen Konstrukt,
wobei das genetische Konstrukt erste und zweite homologe Rekombinationselemente umfasst, die eine Transformationskassette flankieren, wobei die ersten und zweiten homologen Rekombinationselemente dazu in der Lage sind, den Einbau der Transformationskassette in das Genom von mindestens einem Plastid zu steuern, das in der Pflanzenzelle vorhanden ist,
wobei die Transformationskassette Folgendes umfasst:
(a) einen ersten Promotor, der in der Pflanzenzelle funktionsfähig ist,
(b) eine Exzisionskassette,
(c) ein oder mehrere Transgene,
(d) ein erstes Terminatorelement,
wobei die Exzisionskassette Folgendes umfasst:
(b1) ein erstes ortsspezifisches Rekombinationselement,
(b2) einen optionalen zweiten Promotor
(b3) ein oder mehrere Nukleotidsequenzen, die für ein oder mehrere pflanzliche Auxinbiosynthese-Polypeptide kodieren, wobei die pflanzlichen Auxinbiosynthese-Polypeptide ausgewählt werden aus iaaH (Indolacetamidhydrolase) und iaaM (Tryptophanmonooxygenase),
(b4) ein zweites Terminatorelement,
(b5) ein zweites ortsspezifisches Rekombinationselement,
wobei die ersten und zweiten ortsspezifischen Rekombinationselemente lox-Stellen sind und durch eine Rekombinase erkannt werden können, wobei die Rekombinase eine Cre-Rekombinase ist,
vorzugsweise wobei das Verfahren zusätzlich den folgenden Schritt umfasst:
(ii) Selektieren auf transformierte Pflanzenzellen auf Medien, denen Auxin fehlt, und/oder
(iii) Exprimieren einer Rekombinase in der Pflanzenzelle, wobei die Rekombinase dergestalt ist, dass sie die ersten und zweiten ortsspezifischen Rekombinationselemente erkennt.

2. Verfahren nach Anspruch 1:
(A) wobei mindestens eines der ein oder mehreren Transgene für einen Antikörper, ein Antibiotikum, Herbizid, Impfstoff-Antigen, Enzym, einen Enzyminhibitor oder ein Design-Peptid kodiert; und/oder
(B) wobei die Nukleotidsequenzen der homologen Rekombinationselemente derart ausgewählt werden, dass die Transformationskassette spezifisch auf ein oder mehrere ausgewählte Plastide gerichtet wird; und/oder
(C) wobei die Nukleotidsequenzen der homologen Rekombinationselemente derart ausgewählt werden, dass keine oder im Wesentlichen keine Transformationskassetten in das Kerngenom der Pflanze integriert werden; und/oder
(D) wobei die Transformationskassette zusätzlich eine Nukleotidsequenz umfasst, die für ein Polypeptid kodiert, das Resistenz gegenüber einem Antibiotikum verleiht, wobei das Antibiotikum vorzugsweise Spectinomycin ist.

3. Verfahren nach einem der vorherigen Ansprüche, wobei die Pflanze eine Monokotyle oder Dikotyle ist, wobei die Pflanze vorzugsweise ausgewählt wird aus der Gruppe bestehend aus Getreiden (Reis, Weizen, Gerste, Hafer, Sorghum, Mais), Hülsenfrüchten (Luzerne, Linsen, Erdnuss, Erbse, Sojabohne), Ölpflanzen (Palme, Sonnenblume, Kokosnuss, Raps, Olive), Erwerbskulturen (Baumwolle, Zuckerrohr, Maniok), Gemüsepflanzen (Kartoffel, Tomate, Karotte, Süßkartoffel, Zuckerrübe, Kürbis, Gurke, Salat, Brokkoli, Blumenkohl, Stangenbohne, Kohl, Sellerie, Zwiebel, Knoblauch), Obst/ Bäume und Nüsse (Banane, Traube, Cantaloupe-Melone, Zuckermelone, Wassermelone, Erdbeere, Orange, Apfel, Mango, Avocado, Pfirsich, Grapefruit, Ananas, Ahorn, Mandel), Getränke (Kaffee, Tee, Kakao), Holz-Bäume (Eiche, Schwarznuss, Ahorn), Moos und Wasserlinse, besonders bevorzugt, wobei die Pflanze Tabak oder Salat ist.

4. Verfahren nach einem der vorherigen Ansprüche, wobei die Pflanzenzellen Einzelzellen sind, Gruppen von Zellen, in dissoziierter Form oder undissoziierter Form, als Teil eines Pflanzengewebes oder eines Pflanzenteils oder ein Protoplast oder eine Pflanzenflüssigkultur; und/oder wobei die Pflanzenzellen in Pflanzenblättern vorliegen.

5. Verfahren nach einem der vorherigen Ansprüche, wobei das Plastid ausgewählt wird aus Chloroplasten, Leukoplasten, Amyloplasten, Etioplasten, Chromoplasten, Elaioplasten und Gerontoplasten, vorzugsweise, wobei das Plastid ein grünes Plastid ist, besonders bevorzugt wobei das Plastid ein Chloroplast ist.

6. Verfahren zur Herstellung einer Pflanze, welches das Herstellen einer Pflanzenzelle durch ein Verfahren umfasst, wie in einem der vorhergehenden Ansprüche definiert sowie das Regenerieren einer Pflanze aus der Pflanzenzelle.

7. Verfahren zur Herstellung eines Transgenprodukts, das ein Verfahren wie in einem der vorherigen Ansprüche definiert umfasst und zusätzlich den Schritt des Reinigens oder Isolierens und gegebenenfalls Verpackens des Transgenprodukts umfasst, wobei das Transgenprodukt vorzugsweise ein Antikörper ist, ein Antibiotikum, Herbizid, Impfstoff-Antigen, Enzym, Enzyminhibitor oder Design-Peptid.

8. Genetisches Konstrukt, das erste und zweite homologe Rekombinationselemente umfasst, die eine Transformationskassette flankieren, wobei die ersten und zweiten homologen Rekombinationselemente dazu in der Lage sind, den Einbau der Transformationskassette in das Genom von mindestens einem Plastid zu steuern, das in der Pflanzenzelle vorhanden ist, wobei die Transformationskassette Folgendes umfasst:
(a) einen ersten Promotor, der in der Pflanzenzelle funktionsfähig ist,
(b) eine Exzisionskassette,
(c) ein oder mehrere Transgene,
(d) ein erstes Terminatorelement,
wobei die Exzisionskassette Folgendes umfasst:
(b1) ein erstes ortsspezifisches Rekombinationselement,
(b2) einen optionalen zweiten Promotor
(b3) ein oder mehrere Nukleotidsequenzen, die für ein oder mehrere pflanzliche Auxinbiosynthese-Polypeptide kodieren, wobei die pflanzlichen Auxinbiosynthese-Polypeptide ausgewählt werden aus iaaH (Indolacetamidhydrolase) und iaaM (Tryptophanmonooxygenase),
(b4) ein zweites Terminatorelement,
(b5) ein zweites ortsspezifisches Rekombinationselement,
wobei die ersten und zweiten ortsspezifischen Rekombinationelemente von einer Rekombinase erkannt werden können,
wobei die ersten und zweiten ortsspezifischen Rekombinationselemente lox-Stellen sind und wobei die Rekombinase eine Cre-Rekombinase ist.

9. Genetisches Konstrukt nach Anspruch 8:
(A) wobei die Nukleotidsequenzen der homologen Rekombinationselemente derart ausgewählt werden, dass die Transformationskassette spezifisch auf ein oder mehrere ausgewählte Plastide gerichtet wird; oder
(B) wobei die Nukleotidsequenzen der homologen Rekombinationselemente derart ausgewählt werden, dass keine oder im Wesentlichen keine Transformationskassetten in das Kerngenom der Pflanze integriert werden.

10. Pflanzenzelle oder Pflanze oder Pflanzensamen, die/der ein genetisches Konstrukt nach einem der Ansprüche 8 oder 9 umfasst.

11. Verfahren zur Herstellung eines Transgenprodukts, welches das Reinigen oder Isolieren und gegebenenfalls Verpacken eines Transgenprodukts der Pflanzenzelle, Pflanze oder des Pflanzensamens nach Anspruch 10 umfasst, wobei das Transgenprodukt vorzugsweise ein Antikörper ist, ein Antibiotikum, Herbizid, Impfstoff-Antigen, Enzym, Enzyminhibitor oder Design-Peptid.

## Revendications

1. Procédé de production d'une cellule végétale transformée, le procédé comprenant l'étape suivante :
(i) la transformation de la cellule végétale par une construction génétique, dans lequel la construction génétique comprend un premier et un second élément de recombinaison homologues flanquant une cassette de transformation,
dans lequel le premier et le second élément de recombinaison homologues sont capables de commander l'intégration de la cassette de transformation au génome d'au moins un plastide qui est présent dans la cellule végétale,
dans lequel la cassette de transformation comprend :
(a) un premier promoteur qui peut opérer dans ladite cellule végétale,
(b) une cassette d'excision,
(c) un ou plusieurs transgènes,
(d) un premier élément terminateur,
dans lequel la cassette d'excision comprend :
(b1) un premier élément de recombinaison spécifique au site,
(b2) un second promoteur facultatif
(b3) une ou plusieurs séquences nucléotidiques codant pour un ou plusieurs polypeptides biosynthétiques d'auxine végétale, dans lequel les polypeptides biosynthétiques d'auxine végétale sont choisis parmi iaaH (indoléacétamide hydrolase) et iaaM (tryptophane mono-oxygénase),
(b4) un second élément terminateur,
(b5) un second élément de recombinaison spécifique au site,
dans lequel les premier et second éléments de recombinaison spécifiques au site sont des sites lox et sont capables d'être reconnus par une recombinase, dans lequel la recombinase est une recombinase Cre,
de préférence dans lequel le procédé comprend en outre l'étape suivante :
(ii) la sélection des cellules végétales transformées sur des supports qui manquent d'auxine, et/ou
(iii) l'expression d'une recombinase dans la cellule végétale, dans lequel la recombinase est une recombinase qui reconnaît les premier et second éléments de recombinaison spécifiques au site.

2. Procédé selon la revendication 1 :
(A) dans lequel au moins l'un des un ou plusieurs transgènes code pour un anticorps, un antibiotique, un herbicide, un antigène de vaccin, un enzyme, un inhibiteur d'enzyme ou un peptide théorique ; et/ou
(B) dans lequel les séquences nucléotidiques des éléments de recombinaison homologues sont choisies de sorte que la cassette de transformation soit spécifiquement ciblée sur un ou plusieurs plastides choisis ; et/ou
(C) dans lequel les séquences nucléotidiques des éléments de recombinaison homologues sont choisies de sorte qu'aucune ou sensiblement aucune cassette de transformation ne soit intégrée au génome nucléaire de la plante ; et/ou
(D) dans lequel la cassette de transformation comprend en outre une séquence nucléotidique codant pour un polypeptide qui confère une résistance à un antibiotique, de préférence dans lequel l'antibiotique est la spectinomycine.

3. Procédé selon l'une quelconque des revendications précédentes, dans lequel la plante est un monocotylédone ou un dicotylédone, de préférence dans lequel la plante est choisie dans le groupe constitué des céréales (riz, blé, orge, avoine, sorgho, maïs), des légumes (luzerne, lentille, arachide, pois, haricots de soja), des oléagineux (palme, tournesol, coco, canola, olive), des cultures commerciales (coton, canne à sucre, manioc), des récoltes de légumes (pomme de terre, tomate, carotte, patate douce, betterave sucrière, courge, concombre, laitue, brocoli, chou-fleur, haricot sec, chou, céleri, oignon, ail), des fruits, des arbres et des noix (banane, raisin, cantaloup, cantaloup, pastèque, fraise, orange, pomme, mangue, avocat, pêche, raisin, pamplemousse, érable, amande), des boissons (café, thé, cacao), des arbres à bois (chêne, noyer noir, sycomore), de la mousse et des lentilles d'eau, mieux encore dans lequel la plante est le tabac ou la laitue.

4. Procédé selon l'une quelconque des revendications précédentes, dans lequel les cellules végétales sont des cellules individuelles, des groupes de cellules, sous forme dissociée ou sous forme non dissociée, en tant que partie d'un tissu végétal ou d'une pièce végétale, ou d'un protoplaste ou d'une culture végétale liquide ; et/ou dans lequel les cellules végétales sont présentes dans les feuilles des plantes.

5. Procédé selon l'une quelconque des revendications précédentes, dans lequel le plastide est choisi parmi les chloroplastes, les leucoplastes, les amyloplastes, les étioplastes, les chromoplastes, les élaioplastes et les gérontoplastes, de préférence, dans lequel le plastide est un plastide vert, mieux encore dans lequel le plastide est un chloroplaste.

6. Procédé de production d'une plante, comprenant la production d'une cellule végétale par un procédé selon l'une quelconque des revendications précédentes et la régénération d'une plante à partir de la cellule végétale.

7. Procédé de production d'un produit transgénique, comprenant un procédé tel que défini dans l'une quelconque des revendications précédentes et comprenant en outre l'étape de purification ou d'isolement et, éventuellement, d'emballage du produit transgénique, de préférence dans lequel le produit transgénique est un anticorps, un antibiotique, un herbicide, un antigène de vaccin, un enzyme, un inhibiteur d'enzyme ou un peptide théorique.

8. Construction génétique comprenant un premier et un second élément de recombinaison homologues flanquant une cassette de transformation,
dans lequel les premier et second éléments de recombinaison homologues sont capables de commander l'intégration de la cassette de transformation au génome d'au moins un plastide qui est présent dans une cellule végétale,
dans lequel la cassette de transformation comprend :
(a) un premier promoteur qui peut opérer dans ladite cellule végétale,
(b) une cassette d'excision,
(c) un ou plusieurs transgènes,
(d) un premier élément terminateur,
dans lequel la cassette d'excision comprend :
(b1) un premier élément de recombinaison spécifique au site,
(b2) un second promoteur facultatif
(b3) une ou plusieurs séquences nucléotidiques codant pour un ou plusieurs polypeptides biosynthétiques d'auxine végétale, dans lequel les polypeptides biosynthétiques d'auxine végétale sont choisis parmi iaaH (indoléacétamide hydrolase) et iaaM (tryptophane mono-oxygénase),
(b4) un second élément terminateur,
(b5) un second élément de recombinaison spécifique au site,
dans lequel les premier et second éléments de recombinaison spécifiques au site sont capables d'être reconnus par une recombinase,
dans lequel les premier et second éléments de recombinaison spécifiques au site sont des sites lox et dans lequel la recombinase est une recombinase Cre.

9. Construction génétique selon la revendication 8 :
(A) dans lequel les séquences nucléotidiques des éléments de recombinaison homologues sont choisies de sorte que la cassette de transformation soit spécifiquement ciblée sur un ou plusieurs plastides choisis ; ou
(B) dans lequel les séquences nucléotidiques des éléments de recombinaison homologues sont choisies de sorte qu'aucune ou sensiblement aucune cassette de transformation ne s'intègre au génome nucléaire de la plante.

10. Cellule végétale ou plante ou semence de plante comprenant une construction génétique selon l'une quelconque des revendications 8 ou 9.

11. Procédé de production d'un produit transgénique, comprenant la purification ou l'isolement et, éventuellement, l'emballage d'un produit transgénique à partir de la cellule végétale, de la plante ou de la semence de plante de la revendication 10, de préférence dans lequel le produit transgénique est un anticorps, un antibiotique, un herbicide, un antigène de vaccin, un enzyme, un inhibiteur d'enzyme ou un peptide théorique.
